# EUROPEAN PATENT APPLICATION

(11) **EP 4 831 291 A1**
(43) Date of publication of application: **28.10.2026**
(21) Application number: 25736185.7
(22) Date of filing: 02.01.2025
(51) Int. Cl.: C12N 15/85, C12N 15/13, A01K 67/0278, C12N 5/10, C07K 16/28, C07K 16/00

(54) **ANIMAL MODEL FOR PRODUCING HUMANIZED ANTIBODY AND CONSTRUCTION METHOD THEREFOR**

(30) Priority: 02.01.2024 WO PCT/CN2024/070020
(71) Applicant: Gempharmatech Co., Ltd, Nanjing, Jiangsu 210061 (CN)
(72) Inventor: ZHANG, Mingkun, Nanjing, Jiangsu 210061 (CN); ZHANG, Yuxi, Nanjing, Jiangsu 210061 (CN); LIANG, Juan, Nanjing, Jiangsu 210061 (CN); LI, Song, Nanjing, Jiangsu 210061 (CN); ZHAO, Jing, Nanjing, Jiangsu 210061 (CN); GAO, Xiang, Nanjing, Jiangsu 210061 (CN)
(74) Representative: Gong, Jinping
(86) International application number: PCT/CN2025/070157
(87) International publication number: WO 2025/146084

(57) **Abstract**

The present application relates to a non-human animal and a method for preparing the non-human animal. A genome of the non-human animal includes an operably linked human immunoglobulin light chain variable region gene downstream of an immunoglobulin light chain locus, and the human immunoglobulin light chain variable region includes only one human V region gene. The present application further provides a method for producing an antibody through the non-human animal.

## Description

### TECHNICAL FIELD

The present application relates to the field of biomedicine, and in particular to a humanized antibody animal model using a single human light chain V region and a construction method thereof.

### BACKGROUND

Therapeutic monoclonal antibody drugs have undergone more than 30 years of development, have become one of the most important parts of biomedicine, and are currently the most advanced and effective type of drugs that are used increasingly. With the development of this type of drugs, monoclonal antibodies capable of binding to two different antigens or epitopes have been developed, namely bispecific monoclonal antibodies (BsmAb or BsAb). BsAb is an artificially synthesized antibody-like protein that can simultaneously bind to two different antigens or two different epitopes on the same antigen. Namely, bispecific antibodies integrate fragments of antigen binding (Fabs) of two different antibodies. Compared to ordinary antibodies, BsAb can simultaneously bind to two different targets, which not only interferes with different bioactive epitopes physiologically, but also brings two antigens closer in spatial positions physically. BsAb can be designed for various purposes such as increasing targeting specificity, recruiting and activating immune cells, interfering with receptor signaling and inactivating signal ligands, and forcing protein complex binding.

One of the methods that can be used for discovery and screening of humanized BsAb is use of engineered non-human animals carrying human antibody genes. To avoid diversity caused by VJ recombination in light chains, using humanized common light chain mice carrying only a single human light chain for molecular discovery is a low-cost, highly time-efficient method for discovering high-affinity humanized BsAb antibodies. Antibody molecules obtained through the development of common light chain mice have a consistent light chain variable region, and can be used for avoiding non-target heavy/light chain pairing in the production of conventional IgG-like BsAb. This method not only increases the yield from 12.5% to 50%, but also greatly simplifies the purification process of BsAb antibodies. Combined with commonly used techniques such as Knob-in-hole, the production yield of BsAb antibodies can be further increased to 100%.

At present, most of the conventional methods adopted in the industry are to inactivate or delete all encoding regions of light chain variable regions in a non-human animal genome, and insert a functional single rearranged human light chain variable region into the endogenous light chain encoding genome position. However, such solutions often result in gene defect phenotypes caused by large fragment deletions of endogenous genes of the non-human animals. Therefore, there is an urgent need for a new method for constructing a humanized common light chain non-human animal model.

### SUMMARY

The present application provides a non-human animal and a method for preparing the non-human animal. In one aspect, the genome of the non-human animal comprises one human V region gene and a limited number of human J region genes (e.g., 1, 2, 3, 4, or 5 J region genes) downstream of an immunoglobulin light chain locus. The non-human animal, through a complex antibody selection process within the organism, makes biologically appropriate choices when pairing a diverse set of human heavy chain variable domains with a limited number of human light chain variable domain options. The non-human animal is engineered to present a limited number of human light chain variable domain options combined with a wide range of human heavy chain variable domain options. Under the challenge of immunogens, the development of antibodies against the immunogens from the non-human animal is primarily or entirely constrained by the limited of light chain options in the lineage of the non-human animal. In various embodiments, the antibodies produced by the non-human animal have various heavy chains that can bind to the same or substantially the same light chain. When BsAb is prepared, a light chain is associated with a specific heavy chain without the need of antibody engineering modifications (e.g., introducing modifications to the sequence). This can greatly improve the success rate of bispecific antibody development, thereby overcoming the shortcomings caused by a large amount of heavy chain VDJ recombination and light chain VJ recombination mentioned above. In fact, the antibodies or sequences as described herein can further bind to each other to prepare multispecific antibodies.

In another aspect, in the genome of the non-human animal, a human variable region gene (e.g., a light chain variable region, a heavy chain variable region, or a light chain variable region and a heavy chain variable region) is inserted downstream (or the rear) of an endogenous immunoglobulin locus of a non-human animal cell, and at the same time, an endogenous immunoglobulin constant region gene of the non-human animal is inverted (i.e., a transcription direction thereof is rendered to be opposite to that of an endogenous immunoglobulin variable region gene of the non-human animal), which avoids insertion of large-fragment DNA sequences into the endogenous immunoglobulin locus of the non-human animal (e.g., a mouse), or deletion or modification of endogenous DNA sequences, thereby eliminating interference with the expression and regulation of the endogenous genes of the non-human animal, and overcoming the shortcomings caused by the inactivation and deletion of large fragments of the endogenous genes of the non-human animal.

In addition, the non-human animal and the method for preparing the non-human animal in the present application further have some other advantages. For example, in some cases, an inserted exogenous human immunoglobulin variable region is far away from an endogenous variable region and has an opposite transcription direction, thereby avoiding the integration of the endogenous variable region during recombination into the human variable region recombination, and thus avoiding production of antibodies including endogenous immune epitopes.

In one aspect, the present application provides a method for preparing a non-human animal. The method includes operably linking a human immunoglobulin light chain variable region gene downstream of an immunoglobulin light chain locus of the non-human animal. The human immunoglobulin light chain variable region includes only one human V region gene.

In certain implementations, the human immunoglobulin light chain variable region gene further includes one or more human J region genes. In certain implementations, the human immunoglobulin light chain variable region includes 2, 3, 4, or 5 human J region genes. In certain implementations, the one human V region gene and the one or more human J region genes are operably linked.

In certain implementations, the human V region gene is selected from the group consisting of: IGKV4-1, IGKV1-5, IGKV3-11, IGKV3-15, IGKV3-20, IGKV1D-39, IGKV1-39, IGKV2D-28, IGKV1D-33, IGKV1-39, IGKV1-33, IGKV2-30, IGKV2-28, IGKV1-9, IGKV2D-29, IGKV1D-12, IGKV1-12, IGKV1-27, and IGKV1-16. In certain implementations, the human V region gene is selected from the group consisting of: IGKV4-1, IGKV1-5, IGKV3-11, IGKV3-15, IGKV3-20, IGKV1D-39, IGKV1-39, IGKV2D-28, and IGKV1D-33. In certain implementations, the human V region gene and J region gene include a gene rearrangement intron sequence therebetween. In certain implementations, the human V region gene and J region gene do not include the gene rearrangement intron sequence therebetween.

In certain implementations, the human J region gene is selected from the group consisting of: IGKJ5, IGKJ4, IGKJ3, IGKJ2, and IGKJ1. In certain implementations, the human J region gene include the gene rearrangement intron sequence. In certain implementations, the human J region gene does not include the gene rearrangement intron sequence.

In certain implementations, the method includes operably linking a human immunoglobulin heavy chain variable region gene downstream of a heavy chain locus of the non-human animal. In certain implementations, the method includes operably linking one or more genes of human heavy chain V, D, or J regions, or fragments thereof downstream of a heavy chain constant region locus of the non-human animal. In certain implementations, the multiple genes of human heavy chain V, D, or J regions, or fragments thereof may be recombined. In certain implementations, the multiple genes of human heavy chain V, D, or J regions, or fragments thereof are directly linked.

In another aspect, the present application provides a non-human animal or non-human animal cell. A genome of the non-human animal includes an operably linked human immunoglobulin light chain variable region gene downstream of an immunoglobulin light chain locus, and the human immunoglobulin light chain variable region includes only one human V region gene. The features mentioned below that are included in the non-human animal are all included in the non-human animal cell.

In another aspect, the present application further provides a genetically modified non-human animal genome, which includes an operably linked human immunoglobulin light chain variable region gene downstream of an immunoglobulin light chain locus, and the human immunoglobulin light chain variable region includes only one human V region gene.

In certain implementations, the human immunoglobulin light chain variable region gene further includes one or more human J region genes, preferably 2 to 5 human J region genes. In certain implementations, the one human V region gene and the one or more human J region genes are operably linked.

In certain implementations, the human V region gene is selected from the group consisting of: IGKV4-1, IGKV1-5, IGKV3-11, IGKV3-15, IGKV3-20, IGKV1D-39, IGKV1-39, IGKV2D-28, IGKV1D-33, IGKV1-39, IGKV1-33, IGKV2-30, IGKV2-28, IGKV1-9, IGKV2D-29, IGKV1D-12, IGKV1-12, IGKV1-27, and IGKV1-16. In certain implementations, the human V region gene is selected from the group consisting of: IGKV4-1, IGKV1-5, IGKV3-11, IGKV3-15, IGKV3-20, IGKV1D-39, IGKV1-39, IGKV2D-28, and IGKV1D-33. In certain implementations, the human V region gene and J region gene include a gene rearrangement intron sequence therebetween. In certain implementations, the human V region gene and J region gene do not include the gene rearrangement intron sequence therebetween.

In certain implementations, the human J region gene is selected from the group consisting of: IGKJ5, IGKJ4, IGKJ3, IGKJ2, and IGKJ1. In certain implementations, the human J region gene include the gene rearrangement intron sequence. In certain implementations, the human J region gene does not include the gene rearrangement intron sequence.

In certain implementations, the genome of the non-human animal further includes an endogenous immunoglobulin light chain variable region gene. In certain implementations, genomic integrity of an endogenous immunoglobulin light chain variable region remains unchanged. In certain implementations, a function of an expression regulatory element of the endogenous immunoglobulin light chain variable region gene is not disrupted. In certain implementations, the genome of the non-human animal includes a complete expression regulatory element of the endogenous immunoglobulin light chain variable region. In certain implementations, the genome of the non-human animal includes a complete endogenous immunoglobulin light chain variable region gene.

In certain implementations, the non-human animal does not express the endogenous immunoglobulin light chain variable region. In certain implementations, the endogenous immunoglobulin light chain variable region gene of the non-human animal is not expressed as an antibody light chain variable region.

In certain implementations, a transcription direction of the human immunoglobulin light chain variable region gene of the non-human animal is opposite to that of the endogenous immunoglobulin light chain variable region gene. In certain embodiments, the human immunoglobulin light chain variable region gene and the endogenous immunoglobulin light chain variable region include an immunoglobulin light chain constant region gene of the non-human animal therebetween.

In certain embodiments, the transcription direction of the immunoglobulin light chain constant region gene of the non-human animal is opposite to that of the endogenous immunoglobulin light chain variable region gene.

In certain embodiments, the transcription direction of the human immunoglobulin light chain variable region gene of the non-human animal is the same as that of the immunoglobulin light chain constant region gene of the non-human animal.

In certain implementations, the human immunoglobulin light chain variable region gene of the non-human animal and the endogenous immunoglobulin light chain variable region include a light chain constant region gene therebetween. In certain implementations, a distance between the human immunoglobulin light chain variable region gene of the non-human animal and the endogenous immunoglobulin light chain variable region gene is 4 Kbp to 42 Kbp.

In certain implementations, the non-human animal is a rodent. In certain implementations, the non-human animal is a mouse. In certain implementations, the mouse includes the human immunoglobulin light chain variable region gene between lgkc and Rpia genes at a locus of the mouse. In certain implementations, the mouse includes the human immunoglobulin light chain variable region gene between chromosome positions chr6: 70,703,738 and 70,742,704. In certain implementations, the mouse includes the human immunoglobulin light chain variable region gene at a chromosome position chr6: 70,706,267.

In certain implementations, the genome of the non-human animal includes an operably linked human immunoglobulin heavy chain variable region downstream of an immunoglobulin heavy chain locus. In certain implementations, the non-human animal includes one or more operably linked genes of human heavy chain variable region V region, heavy chain variable region D region, or heavy chain variable region J region, or fragments thereof downstream of a heavy chain constant region locus. In certain implementations, the multiple genes of human heavy chain V region, heavy chain variable region D region, or heavy chain variable region J region, or fragments thereof may be recombined. In certain implementations, the multiple genes of human heavy chain variable region V, heavy chain variable region D region, or heavy chain variable region J region, or fragments thereof are directly linked.

In certain implementations, the genome of the non-human animal includes an endogenous immunoglobulin heavy chain variable region gene. In certain implementations, genomic integrity of an endogenous immunoglobulin heavy chain variable region of the non-human animal remains unchanged. In certain implementations, a function of an expression regulatory element of the endogenous immunoglobulin heavy chain variable region gene of the non-human animal is not disrupted. In certain implementations, the genome of the non-human animal includes a complete expression regulatory element of the endogenous immunoglobulin heavy chain variable region. In certain implementations, the genome of the non-human animal includes a complete endogenous immunoglobulin heavy chain variable region gene.

In certain implementations, the non-human animal does not express the endogenous immunoglobulin heavy chain variable region. In certain implementations, the endogenous immunoglobulin variable region gene of the non-human animal is not expressed as an antibody heavy chain variable region.

In certain implementations, the transcription direction of the human immunoglobulin heavy chain variable region gene of the non-human animal is opposite to that of the endogenous immunoglobulin heavy chain variable region gene. In certain implementations, the human immunoglobulin heavy chain variable region gene of the non-human animal and the endogenous immunoglobulin heavy chain variable region include an immunoglobulin heavy chain constant region gene of the non-human animal therebetween.

In certain implementations, the transcription direction of the immunoglobulin heavy chain constant region gene of the non-human animal is opposite to that of the endogenous immunoglobulin heavy chain variable region gene.

In certain implementations, the transcription direction of the human immunoglobulin heavy chain variable region gene of the non-human animal is the same as that of the immunoglobulin heavy chain constant region gene of the non-human animal.

In certain implementations, the human immunoglobulin heavy chain variable region gene of the non-human animal and the endogenous immunoglobulin heavy chain variable region include a heavy chain constant region gene therebetween. In certain implementations, the distance between the human immunoglobulin heavy chain variable region gene of the non-human animal and the endogenous immunoglobulin heavy chain variable region gene is 169 Kbp to 240 Kbp.

In certain implementations, the non-human animal is a rodent. In certain implementations, the non-human animal is a mouse. In certain implementations, the mouse includes the human immunoglobulin heavy chain variable region gene between Tmem121 and Igha genes at a locus of the mouse. In certain implementations, the mouse includes the human immunoglobulin heavy chain variable region gene between chromosome positions chr12: 113,149,523 and 113,223,857. In certain implementations, the human immunoglobulin heavy chain variable region gene is inserted at a chromosome position chr12: 113,190,256 of the mouse.

In another aspect, the present application further provides offspring of the non-human animal. The offspring may be offspring of the non-human animal mating with the same genotype or other genotypes.

In another aspect, the present application further provides cells (e.g., stem cells, embryonic stem cells, immune cells, B cells, T cells, or hybridomas) or cell lines derived from the non-human animal or offspring thereof, or primary cell cultures thereof.

In another aspect, the present application provides an engineered chromosome, which includes the non-human animal genome according to the present application.

In another aspect, the present application provides a non-human animal cell. A genome of the non-human animal cell includes an operably linked human immunoglobulin light chain variable region gene downstream of an immunoglobulin light chain locus, and the human immunoglobulin light chain variable region includes only one human V region gene. In certain implementations, the cell is an embryonic stem (ES) cell. In certain implementations, the cell is a germ cell. In certain implementations, the cell is a fertilized egg cell. In certain implementations, the cell is an embryonic cell.

In certain implementations, the non-human animal cell may develop into a non-human animal.

In another aspect, the present application provides cells, tissues, and organs derived from the non-human animal.

In another aspect, the present application provides a method for preparing an antibody specifically binding to an antigen. The method includes immunizing the non-human animal as described in the present application with the antigen.

In another aspect, the present application provides a method for preparing an antibody specifically binding to an antigen. The method includes: exposing the non-human animal or cell to the antigen, and generating a hybridoma from the cell collected from the animal. The method may further include collecting a chimeric antibody produced by the hybridoma. The method may further include sequencing a variable region gene of the hybridoma.

In another aspect, the present application provides a method for preparing an antibody specifically binding to an antigen. The method includes: exposing the non-human animal or cell to the antigen, and sequencing nucleic acids encoding human heavy chain and light chain immunoglobulin variable regions in the animal or cell. The non-human animal or cell may express a chimeric antibody that specifically binds to the antigen. In one implementation, the method may include operably linking the nucleic acid encoding the human heavy chain immunoglobulin variable region with a nucleic acid encoding a heavy chain immunoglobulin constant region, and expressing the antibody that specifically binds to the antigen. The constant region may be a human heavy chain constant region or a non-human animal heavy chain constant region. In one implementation, the method may include operably linking the nucleic acid encoding the human light chain immunoglobulin variable region with a nucleic acid encoding a light chain immunoglobulin constant region, and expressing the antibody that specifically binds to the antigen. The constant region may be a human light chain constant region or a non-human animal light chain constant region.

In another aspect, the present application provides a method for preparing antibodies specifically binding to two different antigens and having the same or similar light chain variable regions. The method includes: exposing the non-human animal or cell to the two different antigens respectively, and sequencing nucleic acids encoding human heavy chain and light chain immunoglobulin variable regions in the animal or cell. The non-human animal or cells may express a series of antibodies with the same or similar light chain variable regions that specifically bind to different antigens. In one implementation, the method may include operably linking the nucleic acids encoding the human heavy chain immunoglobulin variable regions that specifically bind to the two different antigens and a nucleic acid encoding a heavy chain immunoglobulin constant region respectively, operably linking the same or similar nucleic acid encoding the human light chain immunoglobulin variable region with a nucleic acid encoding a light chain immunoglobulin constant region, and ultimately expressing a bispecific antibody that specifically binds to the two different antigens. The heavy chain immunoglobulin constant region may be a human heavy chain constant region or a non-human animal heavy chain constant region. The light chain immunoglobulin constant region may be a human light chain constant region or a non-human animal light chain constant region.

In some implementations, the antibody is a common light chain antibody. In some embodiments, the common light chain antibody is a common light chain bispecific antibody. In another aspect, the present application provides a method for preparing a sample. The method includes: exposing the non-human animal as described in the present application to an antigen; and collecting the sample from the non-human animal. In certain implementations, the sample includes immune cells, such as B cells. In certain implementations, the sample is derived from bone marrow, spleen tissues, lymph nodes, spleen cells, or peripheral lymphocytes.

The non-human animal as described in the present application may be a rodent, such as a mouse.

The technical solution of the present application includes one or more of the following advantages:
The non-human animal as described in the present application has advantages in avoiding interference with the gene expression and regulation of the animal. For example, a human variable region gene (e.g., a light chain variable region, a heavy chain variable region, or a light chain variable region and a heavy chain variable region) is inserted downstream (or the rear) of the endogenous immunoglobulin locus of the mouse cell to avoid inserting a large-fragment DNA sequence in the endogenous immunoglobulin locus of the non-human animal (e.g., a mouse) or deleting a DNA sequence. Due to the presence of a very long "Junk sequence" downstream of the endogenous immunoglobulin locus of the non-human animal (e.g., the mouse), according to the recently published GRCm39 mouse whole genome sequence reference, a reported sequence of a 66 kb base long non-functional gene exists downstream (or the rear) of the mouse immunoglobulin heavy chain gene, and a reported sequence of a 39 kb base long non-functional gene exists downstream (or the rear) of a mouse immunoglobulin κ light chain gene. Introduction of an exogenous large-fragment gene into these long "Junk sequences" may avoid interfering with the expression and regulation of the endogenous gene of the non-human animal (e.g., the mouse) at the genomic level.

The non-human animal as described in the present application further has advantages in avoiding interference with the gene expression and regulation of the animal. For example, modifying or deleting the immunoglobulin gene variable region of the non-human animal (e.g., the mouse) is avoided. Because there is no lack of functional protein encoding genes and functional microRNA genes within the endogenous immunoglobulin variable region locus of the non-human animal (e.g., the mouse), deletion or modification of these genes may result in unpredictable phenotypes in the animal. For example, the mouse immunoglobulin heavy chain locus includes a functional protein encoding gene Adam6a, and modification or deletion of the Adam6a gene may cause male infertility in the mouse.

The non-human animal as described in the present application has advantages in producing antibodies. In the non-human animal as described in the present application, both the immunoglobulin variable region gene and constant region gene of the host non-human animal are retained, namely, the endogenous immunoglobulin variable region gene and constant region gene of the non-human animal are retained. All host immunoglobulin regulatory sequences, including promoters, enhancers, switch regions, and other potential expression regulatory sequences, are retained. Thereby, the recombination and expression of the immunoglobulin genes, B cell development, affinity maturation, and the like in the non-human animal can be better ensured.

The non-human animal as described in the present application has advantages in producing antibodies. For example, in some embodiments, the animal endogenous immunoglobulin constant region is entirely or partially modified (e.g., the constant region is entirely or partially inverted), so that the transcription direction thereof is opposite to that of the endogenous variable region gene. Thus, V(D)J recombination between the endogenous variable region and the endogenous constant region to produce antibodies is avoided.

The non-human animal as described in the present application further has advantages in producing antibodies. For example, in some embodiments, the inserted exogenous human immunoglobulin variable region is far away from the endogenous variable region and has an opposite transcription direction. Thus, integration of the endogenous variable region into human variable region VDJ recombination or VJ recombination due to DNA recombinase modification and recombination is avoided, and then production of antibodies with immunogenic epitopes in humans caused by an endogenous variable region lineage is highly possibly avoided.

The non-human animal as described in the present application further has advantages in producing antibodies. For example, in some embodiments, by gradually introducing and inserting a limited number of human variable region gene fragments (including all human IGK J regions and a small number of human IGK V regions) in batches downstream (or the rear) of the endogenous immunoglobulin locus of the non-human animal (e.g., the mouse) cell, the obtained variable domain produced by the non-human animal may have limited light chain diversity, and the diversity of light chains caused by VJ recombination can be avoided.

The non-human animal as described in the present application further has advantages in producing antibodies. For example, in some embodiments, the present application avoids the problem of different usage degrees of the V region. The frequencies of usage of the human immunoglobulin light chain variable V regions have differences under natural conditions, which may be due to the physical arrangement of the spatial distance among the V region, the J region, and the C region in the human genome. The strategy of the present application uses a single IGKV, which avoids the problem of the usage degree of the light chain V regions, so that all inserted human IGKVs can be used in almost all B cells of the living biological system of the mouse, and a sufficient expression level is obtained. In some embodiments, the expression may not be affected by a low frequency of usage of the V region in nature, and the present application can be used for developing an antibody using a low-frequency IGKV, and for example, has unique advantages in the development of antibody drugs for some emerging epidemic viruses.

The method for preparing a non-human animal as described in the present application also has advantages. For example, in some embodiments, the endogenous immunoglobulin constant region of the non-human animal (e.g., the mouse) is entirely or partially modified, so that the transcription direction thereof is opposite to that of the endogenous variable region gene. Then, the modified human variable region gene is inserted downstream (the rear) of the endogenous immunoglobulin locus of the non-human animal (e.g., the mouse) cell. The human variable region gene may be segmented from rear to front and inserted in an opposite direction to transcription of the animal immunoglobulin locus. The obtained effect is that almost every segment of modified cells inserted into the obtained part of the human variable region may be injected into an early animal embryo to prepare a chimeric animal (e.g., a mouse), so as to produce or obtain the non-human animal through breeding. The obtained non-human animals can utilize the inserted part of the human variable region gene for VDJ or VJ recombination, and produce a chimeric antibody of the functional human variable region. These non-human animals carrying partial human variable regions may serve as check points for producing non-human animals with complete human variable regions. The reliability and functionality of segmented insertion of the human variable regions are determined from the perspective of antibody production and diversity of variable region domains, so as to make timely prediction of success or failure and route correction for production of non-human animals with complete human variable regions. These non-human animals carrying partial human variable regions may also be discovered with antibodies for the production of human variable region chimeric antibodies.

The method for preparing a non-human animal as described in the present application also has advantages. For example, in some embodiments, the method of the present application can be used for rapidly preparing a series of non-human animals carrying different human light chain variable region V genes. In some embodiments, inversion of the endogenous constant regions of non-human animals is first performed to obtain intermediates (cells or animals), and then different single human IGK V regions are inserted downstream of the endogenous immunoglobulin loci of the intermediates to obtain a series of non-human animals carrying different human light chain variable region V genes. By performing antibody discovery in non-human animals carrying different human light chain variable region V genes, it is possible to minimize the situation where high-affinity antibodies cannot be obtained for certain targets due to the lack of diversity in the IGK V region. At the same time, a variety of different single human IGK V region animals can be used for discovering antibodies against some universal targets of multi-antibody drugs (e.g., CD3 and CD16A), and establishing universal shelf-style antibody molecules to match other targets with increasing diversity.

The present application further provides the following embodiments:
The present application provides a method for preparing a non-human animal. The method includes operably linking a human immunoglobulin light chain variable region gene downstream of an immunoglobulin light chain locus of the non-human animal. The human immunoglobulin light chain variable region includes only one human V region gene.

In some implementations, the human immunoglobulin light chain variable region gene further includes one or more human J region genes.

In some implementations, the human immunoglobulin light chain variable region includes 2 to 5 human J region genes.

In some implementations, the one human V region gene and the one or more human J region genes are operably linked.

In some implementations, the human V region gene is selected from the group consisting of: IGKV4-1, IGKV1-5, IGKV3-11, IGKV3-15, IGKV3-20, IGKV1D-39, IGKV1-39, IGKV2D-28, and IGKV1D-33.

In some implementations, the human V region gene and J region gene include a gene rearrangement intron sequence.

In some implementations, the human V region gene and J region gene do not include the gene rearrangement intron sequence.

In some implementations, the human J region gene is selected from the group consisting of: IGKJ5, IGKJ4, IGKJ3, IGKJ2, and IGKJ1.

In some implementations, the human J region gene includes the gene rearrangement intron sequence.

In some implementations, the human J region gene does not include the gene rearrangement intron sequence.

In some implementations, the non-human animal includes an endogenous immunoglobulin light chain variable region gene.

In some implementations, genomic integrity of an endogenous immunoglobulin light chain variable region of the non-human animal remains unchanged.

In some implementations, the function of an expression regulatory element of the endogenous immunoglobulin light chain variable region gene of the non-human animal is not disrupted.

In some implementations, the non-human animal includes a complete expression regulatory element of the endogenous immunoglobulin light chain variable region.

In some implementations, the non-human animal includes a complete endogenous immunoglobulin light chain variable region gene.

In some implementations, the non-human animal does not express the endogenous immunoglobulin light chain variable region.

In some implementations, the endogenous immunoglobulin light chain variable region gene of the non-human animal is not expressed as an antibody light chain variable region.

In some implementations, the method includes rendering the transcription direction of the human immunoglobulin light chain variable region gene to be opposite to that of the endogenous immunoglobulin light chain variable region gene.

In some implementations, the human immunoglobulin light chain variable region gene and the endogenous immunoglobulin light chain variable region include an immunoglobulin light chain constant region gene of the non-human animal therebetween.

In some implementations, the method includes rendering the transcription direction of the immunoglobulin light chain constant region gene of the non-human animal to be opposite to that of the endogenous immunoglobulin light chain variable region gene.

In some implementations, in the non-human animal, the transcription direction of the human immunoglobulin light chain variable region gene is the same as that of the immunoglobulin light chain constant region gene of the non-human animal.

In certain implementations, the human immunoglobulin light chain variable region gene of the non-human animal and the endogenous immunoglobulin light chain variable region include a light chain constant region gene therebetween. In some implementations, a distance between the human immunoglobulin light chain variable region gene and the endogenous immunoglobulin light chain variable region gene is 4 Kbp to 42 Kbp.

In some implementations, the non-human animal is a rodent.

In some implementations, the non-human animal is a mouse.

In some implementations, the method includes inserting the human immunoglobulin light chain variable region gene between lgkc and Rpia genes at a locus of the mouse.

In some implementations, the method includes inserting the human immunoglobulin light chain variable region gene between chromosome positions chr6: 70,703,738 and 70,742,704 of the mouse.

In some implementations, the method includes inserting the human immunoglobulin light chain variable region gene at a chromosome position chr6: 70,706,267 of the mouse.

In some implementations, the method includes enabling the human immunoglobulin light chain variable region gene to be operably linked downstream of the immunoglobulin locus of the non-human animal through site-directed recombination.

In some implementations, the method includes rendering the transcription direction of the immunoglobulin light chain constant region gene of the non-human animal to be opposite to that of the endogenous immunoglobulin light chain variable region gene through genome modification.

In some implementations, the method further includes operably linking a human immunoglobulin heavy chain variable region gene downstream of the immunoglobulin heavy chain locus of the non-human animal.

In some implementations, the method includes operably linking one or more genes of human heavy chain variable region V region, heavy chain variable region D region, or heavy chain variable region J region, or fragments thereof downstream of a heavy chain constant region locus of the non-human animal.

In some implementations, the multiple genes of human heavy chain variable region V region, heavy chain variable region D region, or heavy chain variable region J region, or fragments thereof are directly linked.

In some implementations, the non-human animal includes an endogenous immunoglobulin heavy chain variable region gene.

In some implementations, genomic integrity of an endogenous immunoglobulin heavy chain variable region of the non-human animal remains unchanged.

In some implementations, a function of an expression regulatory element of the endogenous immunoglobulin heavy chain variable region gene of the non-human animal is not disrupted.

In some implementations, the non-human animal includes a complete expression regulatory element of the endogenous immunoglobulin heavy chain variable region.

In some implementations, the non-human animal includes a complete endogenous immunoglobulin heavy chain variable region gene.

In some implementations, the non-human animal does not express the endogenous immunoglobulin heavy chain variable region.

In some implementations, the endogenous immunoglobulin variable region gene of the non-human animal is not expressed as an antibody heavy chain variable region.

In some implementations, the method includes rendering the transcription direction of the human immunoglobulin heavy chain variable region gene to be opposite to that of the endogenous immunoglobulin heavy chain variable region gene.

In some implementations, the human immunoglobulin heavy chain variable region gene of the non-human animal and the endogenous immunoglobulin heavy chain variable region include an immunoglobulin heavy chain constant region gene of the non-human animal therebetween.

In some implementations, the method includes rendering the transcription direction of the immunoglobulin heavy chain constant region gene of the non-human animal to be opposite to that of the endogenous immunoglobulin heavy chain variable region gene.

In some implementations, the transcription direction of the human immunoglobulin heavy chain variable region gene of the non-human animal is the same as that of the immunoglobulin heavy chain constant region gene of the non-human animal.

In certain implementations, the human immunoglobulin heavy chain variable region gene of the non-human animal and the endogenous immunoglobulin heavy chain variable region include a heavy chain constant region gene therebetween. In some implementations, a distance between the human immunoglobulin heavy chain variable region gene of the non-human animal and the endogenous immunoglobulin heavy chain variable region gene is 169 Kbp to 240 Kbp.

In some implementations, the method includes inserting the human immunoglobulin heavy chain variable region gene between Tmem121 and Igha genes at a locus of the mouse.

In some implementations, the method includes inserting the human immunoglobulin heavy chain variable region gene between chromosome positions chr12: 113,149,523 and 113,223,857 of the mouse.

In some implementations, the method includes inserting the human immunoglobulin heavy chain variable region gene at a chromosome position chr12: 113,190,256 of the mouse.

In some implementations, the method includes enabling the human immunoglobulin variable region gene to be operably linked downstream of the immunoglobulin locus of the non-human animal through site-directed recombination.

In some implementations, the method includes rendering the transcription direction of an immunoglobulin constant region gene of the non-human animal to be opposite to that of the endogenous immunoglobulin variable region gene through genome modification.

The present application provides a non-human animal, and a genome of the non-human animal includes an operably linked human immunoglobulin light chain variable region gene downstream of an immunoglobulin light chain locus, and the human immunoglobulin light chain variable region includes only one human V region gene.

In some implementations, the human immunoglobulin light chain variable region gene further includes one or more human J region genes, preferably 2 to 5 human J region genes.

In some implementations, the one human V region gene and the one or more human J region genes are operably linked.

In some implementations, the human V region gene is selected from the group consisting of: IGKV4-1, IGKV1-5, IGKV3-11, IGKV3-15, IGKV3-20, IGKV1D-39, IGKV1-39, IGKV2D-28, IGKV1D-33.

In some implementations, the human V region gene and J region gene include a gene rearrangement intron sequence therebetween.

In some implementations, the human V region gene and J region gene do not include the gene rearrangement intron sequence therebetween.

In some implementations, the human J region gene is selected from the group consisting of: IGKJ5, IGKJ4, IGKJ3, IGKJ2, and IGKJ1.

In some implementations, the human J region gene include the gene rearrangement intron sequence.

In some implementations, the human J region gene does not include the gene rearrangement intron sequence.

In some implementations, the genome of the non-human animal further includes an endogenous immunoglobulin light chain variable region gene.

In some implementations, genomic integrity of an endogenous immunoglobulin light chain variable region remains unchanged.

In some implementations, a function of an expression regulatory element of the endogenous immunoglobulin light chain variable region gene is not disrupted.

In some implementations, the genome of the non-human animal includes a complete expression regulatory element of the endogenous immunoglobulin light chain variable region.

In some implementations, the genome of the non-human animal includes a complete endogenous immunoglobulin light chain variable region gene.

In some implementations, the non-human animal does not express the endogenous immunoglobulin light chain variable region.

In some implementations, the endogenous immunoglobulin light chain variable region gene is not expressed as an antibody light chain variable region.

In some implementations, a transcription direction of the human immunoglobulin light chain variable region gene is opposite to that of the endogenous immunoglobulin light chain variable region gene.

In some implementations, the human immunoglobulin light chain variable region gene and the endogenous immunoglobulin light chain variable region include an immunoglobulin light chain constant region gene of the non-human animal therebetween.

In some implementations, the transcription direction of the immunoglobulin light chain constant region gene is opposite to that of the endogenous immunoglobulin light chain variable region gene.

In some implementations, the transcription direction of the human immunoglobulin light chain variable region gene of the non-human animal is the same as that of the immunoglobulin constant region gene of the non-human animal.

In some implementations, the human immunoglobulin light chain variable region gene of the non-human animal and the endogenous immunoglobulin light chain variable region include a light chain constant region gene therebetween. In some implementations, a distance between the human immunoglobulin light chain variable region gene and the endogenous immunoglobulin light chain variable region gene is 4 Kbp to 42 Kbp.

In some implementations, the non-human animal is a rodent.

In some implementations, the non-human animal is a mouse.

In some implementations, the non-human animal includes the human immunoglobulin light chain variable region gene between lgkc and Rpia genes at a locus of the mouse.

In some implementations, the non-human animal includes the human immunoglobulin light chain variable region gene between chromosome positions chr6: 70,703,738 and 70,742,704.

In some implementations, the non-human animal includes the human immunoglobulin light chain variable region gene at a chromosome position chr6: 70,706,267.

In some implementations, the genome of the non-human animal includes an operably linked human immunoglobulin heavy chain variable region downstream of an immunoglobulin heavy chain locus.

In some implementations, the gene located downstream of a heavy chain constant region locus includes one or more operably linked genes of human heavy chain variable region V region, heavy chain variable region D region, or heavy chain variable region J region, or fragments thereof.

In some implementations, the multiple genes of human heavy chain variable region V region, heavy chain variable region D region, or heavy chain variable region J region, or fragments thereof are directly linked.

In some implementations, the genome of the non-human animal includes an endogenous immunoglobulin heavy chain variable region gene.

In some implementations, genomic integrity of an endogenous immunoglobulin heavy chain variable region remains unchanged.

In some implementations, a function of an expression regulatory element of the endogenous immunoglobulin heavy chain variable region gene is not disrupted.

In some implementations, the genome of the non-human animal includes a complete expression regulatory element of the endogenous immunoglobulin heavy chain variable region.

In some implementations, the genome of the non-human animal includes a complete endogenous immunoglobulin heavy chain variable region gene.

In some implementations, the non-human animal does not express the endogenous immunoglobulin heavy chain variable region.

In some implementations, the endogenous immunoglobulin variable region gene is not expressed as an antibody heavy chain variable region.

In some implementations, the transcription direction of the human immunoglobulin heavy chain variable region gene is opposite to that of the endogenous immunoglobulin heavy chain variable region gene.

In some implementations, the human immunoglobulin heavy chain variable region gene and the endogenous immunoglobulin heavy chain variable region include an immunoglobulin heavy chain constant region gene of the non-human animal therebetween.

In some implementations, the transcription direction of the immunoglobulin heavy chain constant region gene of the non-human animal is opposite to that of the endogenous immunoglobulin heavy chain variable region gene.

In some implementations, the transcription direction of the human immunoglobulin heavy chain variable region gene is the same as that of the immunoglobulin heavy chain constant region gene of the non-human animal.

In some implementations, the human immunoglobulin heavy chain variable region gene of the non-human animal and the endogenous immunoglobulin heavy chain variable region include a heavy chain constant region gene therebetween. In some implementations, the distance between the human immunoglobulin heavy chain variable region gene and the endogenous immunoglobulin heavy chain variable region gene is 169 Kbp to 240 Kbp.

In some implementations, the non-human animal is a rodent, and the human immunoglobulin heavy chain variable region gene is included between Tmem121 and Igha genes at a locus of the mouse.

In some implementations, the non-human animal is a rodent, the human immunoglobulin heavy chain variable region gene is included between chromosome positions chr12: 113,149,523 and 113,223,857.

In some implementations, the non-human animal is a rodent, the human immunoglobulin heavy chain variable region gene is inserted at a chromosome position chr12: 113,190,256.

Those skilled in the art can easily perceive other aspects and advantages of the present application from the following detailed description. In the following detailed description, only exemplary embodiments of the present application are shown and described. It will be recognized by those skilled in the art that changes can be made to the disclosed specific embodiments by those skilled in the art based on the disclosure of the present application without departing from the spirit and scope of the present application. Accordingly, correspondingly, the drawings and descriptions in the specification of the present application are only exemplary rather than restrictive.

### BRIEF DESCRIPTION OF THE DRAWINGS

Specific features of the invention to which the present application pertains are shown in the appended claims. The features and advantages of the invention to which the present application pertains can be better understood by referring to the exemplary embodiments and drawings described in detail below. A brief description of the attached drawings is as follows:
FIG. 1 shows an exemplary IgK constant region Cpoint and LD targeting as well as C-region modification solution.
FIG. 2 shows a schematic diagram of inserting a light chain human variable region, including IGK-vector targeting and resistance marker deletion.
FIG. 3 shows an exemplary IgH constant region Cpoint and LD targeting as well as C-region modification solution.
FIG. 4 shows a schematic diagram of inserting a heavy chain human variable region, including IGH-vector targeting and resistance marker deletion.
FIG. 5 shows PCR identification results of Cpoint modification of a light chain.
FIG. 6 shows PCR identification results of Cpoint modification of a heavy chain.
FIG. 7 shows LD PCR identification results of a light chain.
FIG. 8 shows LD PCR identification results of a heavy chain.
FIG. 9 shows PCR identification results of inversion of a light chain constant region.
FIG. 10 shows PCR identification results of inversion of a heavy chain constant region.
FIG. 11 shows identification results of deletion of a light chain resistance marker.
FIG. 12 shows identification results of deletion of a heavy chain resistance marker.
FIG. 13A and FIG. 13B show identification results of IGK VJ insertion of a light chain.
FIG. 14A and FIG. 14B show PCR identification results of IGH vector1 targeting and integrity.
FIG. 15 shows PCR identification results of deletion of an IGH vector1 resistance marker.
FIG. 16 shows the frequency of usage of a V region of a human immunoglobulin light chain (IGK).
FIG. 17 shows the frequency of usage of a J region of a human immunoglobulin light chain (IGK).
FIG. 18 shows two feasible strategies for exemplary IGK V and IGK J linkage in the present application.
FIG. 19 shows the usage counts of light chain variable regions V region and J region in BCR sequencing of a humanized mouse.
FIG. 20 shows that a human IGK variable region V region of a humanized mouse is used almost 100% in all B cells.
FIG. 21 shows that a human IGK variable region J region of a humanized mouse is used almost 100% in all B cells.
FIG. 22 shows development of spleen immune cells of a humanized mouse.
FIG. 23 shows development of myeloid immune cells of a humanized mouse.
FIG. 24 shows affinity analysis results of 12 Unique antibody molecules.
FIG. 25 shows Ec50 data of Unique antibody molecules.
FIG. 26 shows analysis of yeast enrichment of a human-derived antigen at different concentrations using FACS.
FIG. 27 shows FACS level affinity detection of antibodies in different clone supernatants.
FIG. 28 shows BCR sequencing results of humanized mouse IGH.
FIG. 29 shows BCR sequencing results of humanized mouse IGK.
FIG. 30 shows ELISA titer detection results of a humanized mouse serum antibody.

### DETAILED DESCRIPTION

The implementations of the present application will be described by way of specific embodiments, and those skilled in the art can easily understand other advantages and effects of the present application from the disclosure in the present specification.

### DEFINITION OF TERMS

In the present application, the term "non-human animal" generally refers to all non-human vertebrates, for example mammals and non-mammals, such as non-human primates, rodents, rabbits, sheep, dogs, cats, horses, cows, birds, amphibians and reptiles. For example, the non-human animal can be a rat or a mouse.

In the present application, the term "constant region" generally refers to a sum of domains of an antibody except variable regions. The constant region does not directly involve the binding of an antigen, but shows different effector functions. Depending on the amino acid sequences of the constant regions of their heavy chains, the antibodies are classified as the following categories: IgA, IgD, IgE, IgG and IgM, and some of them can be further classified as categories IgG1, IgG2 and IgG3, as well as IgG4, IgA1 and IgA2. The heavy chain constant regions corresponding to different categories of antibodies are referred to as α, δ, ε, γ and µ, respectively. The light chain constant regions found in all of 5 categories of antibodies are referred to as κ (kappa) and λ (lambda). The genes encoding mouse (Mus musculus) constant regions can comprise IGHA, IGHD, IGHE, IGHG1, IGHG2A, IGHG2B, IGHG2C, IGHG3 or IGHM. Information about mouse constant region loci can see IMGT Repertoire:https://www.imgt.org/IMGTrepertoire/index.php?section=LocusGenes&repertoire=ge netable&species=Mus_musculus&group=IGHC.

In the present application, the term "variable region (antibody variable region)" typically refers to a region where an antibody molecule binds to a specific antigen. An antibody variable region consists of antigen binding sites for a heavy chain and a light chain, and is divided into a heavy chain variable region and a light chain variable region, respectively. According to a gene encoding exon, the variable region may be further subdivided into a V (variable) region, a D (diversity) region, and a J (joining) region, where the light chain variable region only includes a V (variable) region and a J (joining) region. The variable regions are different between different B cell immunoglobulins, but are the same between all the immunoglobulins produced by the same B cell. The diversity of the variable region is generated by gene recombination of variable region genes during the B cell maturation. This process is a rearrangement process which generates lots of diversity capable of binding to any given antigens, and therefore an immune system recognizes and neutralizes a large amount of antigenic burdens caused by exogenous and pathogenic structures. Therefore, an antibody library is composed of rich immunoglobulins having different V regions, but the immunoglobulins have the same Fc portion.

In the present application, the term "locus" generally refers to a specific region along a chromosome or a DNA sequence. Depending on the contexts, the locus can be a specific sequence of a gene, a marker, a chromosome band, or one or more nucleotides. In the present application, when it comes to the immunoglobulin locus, the locus comprises a genetic element or a set of related genetic elements whose cells can be used for expressing the information of an immunoglobulin peptide. As to a locus that is not rearranged, the genetic element can be assembled by a B cell precursor to form a gene encoding the immunoglobulin peptide. As to a locus that is rearranged, the gene encoding the immunoglobulin peptide is contained in the locus.

In the present application, the term "antibody" generally refers to a support or skeleton portion comprising an intact antibody or an antigen binding fragment thereof. And optionally allowing a portion binding to the antigen to adopt a conformation promoting the binding of the antibody to the antigen. The examples of the antibody include but are not limited to a monoclonal antibody, a polyclonal antibody, Fab, Fab', F(ab)2, a Fv fragment, F(ab')2, scFv, di-scFv and/or dAb, an immunoconjugate, a multi-specific antibody (for example a bispecific antibody), an antibody fragment, an antibody derivative, an antibody analogue or a fusion protein and the like, as long as they show required antigen binding activity. The term also includes a genetically engineered antibody such as a chimeric antibody (for example a humanized mouse antibody), a humanized antibody, a fully human antibody and a heterologous covalent antibody (for example a bispecific antibody).

In the present application, "immunoglobulin" generally refers to a protein essentially consisting of one or more polypeptides encoded by immunoglobulin genes. The recognized human immunoglobulin genes comprise κ, λ, α (IgA1 and IgA2), γ (IgG1, IgG2, IgG3, and IgG4), δ, ε and µ constant region genes, as well as numerous immunoglobulin variable region genes. The NH2- terminal (about 110 amino acids) of a full-length immunoglobulin "light chain" (about 25 KD and 214 amino acids) is encoded by variable region genes, and the COOH- terminal is encoded by κ or λ constant region genes. The full-length immunoglobulin "heavy chain" (about 50 KD and 446 amino acids) is encoded similarly by variable region gene (about 116 amino acids) and one of the above other constant region genes such as γ (encoding about 330 amino acids). The term "immunoglobulin" comprises immunoglobulins of CDR from human or non-human sources. The structure of the immunoglobulin can be human, humanized or non-human, for example a mouse structure with reduced antigenicity in a human body via modification, or a synthesized structure for example a consensus sequence.

In the present application, the term "endogeneity" generally refers to any substance derived from or within organisms, cells, tissues, or systems. For example, an endogenous gene, i.e., a gene naturally occurring in a non-human animal, is different from an exogenous gene that is from other animals, and introduced through transgenosis.

In the present application, the terms "downstream" is a generally description of relative positions between genes in a chromosome, with reference to the chromosome, or a given gene. In the present application, to better conclude modification common points of light chain and heavy chain loci, with reference to natural immunoglobulin locus position of a non-human animal host and its immunoglobulin transcription direction (i.e., as shown in NCBI database), for example, for gene A, if the position of the gene A is the rear of the coding terminal of the immunoglobulin locus relative to the host immunoglobulin locus, it is considered that A is located downstream of the host immunoglobulin locus.

In the present application, the term "transcription direction" generally refers to a gene coding direction where genes finally form a functional protein, i.e., a direction from a sequence of a coding initiation codon (such as ATG) to a sequence of a coding termination codon (such as TAG, TAA and TGA).

In the present application, the term "gene rearrangement intron" typically refers to a DNA sequence that plays a role in DNA rearrangement of an immunoglobulin variable region, and is typically not transcribed into RNA or expressed as a protein or peptide. A gene rearrangement intron may include a rearrangement signal sequence (RSS), which is a segment of sequence located at two ends of the V, D, and J fragments in an Ig germline gene, and includes a 7-nucleotide sequence (CACAGTG) with a palindromic feature and an A-rich 9-nucleotide sequence (ACAAAAACC), along with a 12 or 23 bp spacer sequence therebetween. For example, before gene rearrangement, the intron sequence between the V region and the J region of the light chain variable region includes an RSS sequence, while after gene rearrangement, the V region and the J region of the light chain variable region do not contain the RSS sequence in a VJ intron.

### DETAILED DESCRIPTION OF THE INVENTION

### Non-human animals or cells

The present application relates to a genetically modified non-human animal and cell capable of producing a chimeric antibody with a human variable region and having a functional human immunoglobulin variable region gene. In the present application, the research conducted in mice is only an example. Unless otherwise specified, all non-human mammals are also included in the reference to mice, and mice are preferred non-human mammals.

To obtain a non-human animal that produces an antibody with a human variable region, a human variable region gene (e.g., a light chain variable region, a heavy chain variable region, or a light chain variable region and a heavy chain variable region) is operably linked downstream of an endogenous immunoglobulin locus of a non-human animal cell, and at the same time, in some embodiments, an endogenous immunoglobulin constant region of the non-human animal is entirely or partially modified (e.g., the constant region is entirely or partially inverted), so that the transcription direction thereof is opposite to that of an endogenous variable region gene. Thus, while retaining the immunoglobulin variable region gene and constant region gene of the non-human animal, expression of an endogenous variable region of the non-human animal can be avoided. Moreover, the transcription direction of the endogenous variable region gene of the non-human animal can be rendered to be opposite to those of the endogenous constant region and the human variable region gene of the non-human animal, thereby avoiding production of antibodies due to V(D)J recombination between the endogenous variable region and the endogenous constant region, avoiding integration of the endogenous variable region into the human variable region, and further avoiding production of antibodies with immunogenic epitopes in humans derived from the endogenous variable region lineage.

In some implementations, the human variable region gene of the non-human animal genome includes only one IGK V region gene and a limited number of IGK J region genes, for example, 1, 2, 3, 4, or 5 IGK J region genes. Therefore, the diverse antibodies produced by the non-human animal have diverse heavy chains that can bind to the same or substantially the same light chain. This characteristic is particularly useful in preparation of a bispecific antibody (BsAb). For example, such a non-human animal may induce production of B cells by immunization with a first antigen, and antibodies expressed by these B cells can specifically bind to the first antigen. Similarly, the non-human animal (or an animal with the same modification) may also produce B cells capable of expressing antibodies that can specifically bind to a second antigen through immunization with the second antigen. Subsequently, corresponding VHs (heavy chain variable regions) may be cloned from the first and second types of B cells, respectively. These two VHs may be paired with the same light chain VL (light chain variable region) to prepare a bispecific antibody. Therefore, an antibody engineering method (e.g., modifying sequences) is not needed to forcibly pair certain light chains with a specific heavy chain. This method significantly improves the success rate of bispecific antibody development. In addition, the antibodies or related sequences thereof as described herein may be further combined with each other for preparing multispecific antibodies, for example, bispecific antibodies (BsAb).

A κ chain immunoglobulin locus (also known as IGK or immunoglobulin κ locus) is a region that contains a human antibody (or immunoglobulin) light chain gene on a chromosome (e.g., human chromosome 2). The immunoglobulin light chain gene may undergo a series of rearrangements, leading to production of a mature immunoglobulin light chain nucleic acid (e.g., a κ chain).

The linkage between a V region of the κ chain immunoglobulin locus (also known as an IGKV gene) and a J region of the κ chain immunoglobulin locus (also known as an IGKJ gene) produces a continuous exon, and the exon encodes an entire light chain variable domain. In a non-rearranged DNA, a V gene segment (or an IGKV gene cluster) is located relatively far from a constant (C) region. A J gene segment (or an IGKJ gene cluster) is located near the C region. The linkage between the V region and the J region also makes the V gene approach a constant region sequence. The rearranged V and J gene segments are separated from the C region sequence by only one intron. To produce a complete immunoglobulin light chain messenger RNA, after transcription, introns are removed through RNA splicing to directly link a V region exon, a J region exon, and a C region exon in sequence.

The human light chain immunoglobulin locus is located on the human chromosome 2. Human IGKV genes are divided into several subgroups, including IGKV1 genes (including all IGKV genes starting from IGKV1, also known as VκI), IGKV2 genes (including all IGKV genes starting from IGKV2, also known as VκII), IGKV3 genes (including all IGKV genes starting from IGKV3, also known as VκIII), IGKV4 genes (including all IGKV genes starting from IGKV4, also known as VκIV), IGKV5 genes (including all IGKV genes starting from IGKV5, also known as VκV), IGKV6 genes (including all IGKV genes starting from IGKV6, also known as VκVI), and IGKV7 genes (including all IGKV genes starting from IGKV7, also known as VκVII). In addition, the IGKV genes in the human chromosome 2 also form two clusters, namely a proximal Vκ cluster and a distal Vκ cluster, with similar but different sequences in the two clusters. The human IGKJ genes include IGKJ1, IGKJ2, IGKJ3, IGKJ4, and IGKJ5.

For information on the human light chain V region and J region, as well as the heavy chain V region, D region, and J region, please refer to IMGT Repertoire: https://www.imgt.org/IMGTrepertoire/LocusGenes/.

In one aspect, the non-human animal or cell as described in the present application has one human light chain (e.g., IGK) V region and one or more human light chain (e.g., IGK) J regions downstream of an encoding region of a light chain locus of the host non-human animal, for example, including one human light chain (e.g., IGK) V region and 1, 2, 3, 4, or 5 human light chain (e.g., IGK) J regions. In some embodiments, transcription directions of encoding regions of the human light chain (e.g., IGK) V region and the human light chain (e.g., IGK) J region after insertion are opposite to those of corresponding endogenous light chain VJ original gene encoding regions of the host. The human IGK V region and the human IGK J region are operably linked together and may undergo VJ recombination.

In some embodiments, a constant region of an animal endogenous light chain locus is entirely or partially modified, so that the transcription direction thereof is completely or partially opposite to that of the corresponding endogenous light chain variable region gene.

In some embodiments, the human V region gene is IGKV4-1, IGKV1-5, IGKV3-11, IGKV3-15, IGKV3-20, IGKV1D-39, IGKV1-39, IGKV2D-28, IGKV1D-33, IGKV 1-39, IGKV1-33, IGKV2-30, IGKV2-28, IGKV1-9, IGKV2D-29, IGKV1D-12, IGKV1-12, IGKV1-27, or IGKV1-16. In some embodiments, the human V region gene is IGKV4-1, IGKV1-5, IGKV3-11, IGKV3-15, IGKV3-20, IGKV1D-39, IGKV1-39, IGKV2D-28, or IGKV1D-33.

In some embodiments, the human J region gene is selected from 1 to 5 of the group consisting of: IGKJ5, IGKJ4, IGKJ3, IGKJ2, and IGKJ1.

In some embodiments, the human V region gene is selected from one of the group consisting of: IGKV4-1, IGKV1-5, IGKV3-11, IGKV3-15, IGKV3-20, IGKV1D-39, IGKV1-39, IGKV2D-28, IGKV1D-33, IGKV1-39, IGKV1-33, IGKV2-30, IGKV2-28, IGKV1-9, IGKV2D-29, IGKV1D-12, IGKV1-12, IGKV1-27, and IGKV1-16. At the same time, the human J region gene is selected from 1 to 5 of the group consisting of: IGKJ5, IGKJ4, IGKJ3, IGKJ2, and IGKJ1. For example, when the human V region gene is IGKV4-1, the human J region gene is 1, 2, 3, 4, or 5 of IGKJ5, IGKJ4, IGKJ3, IGKJ2, and IGKJ1. When the human V region gene is IGKV1-5, the human J region gene is 1, 2, 3, 4, or 5 of IGKJ5, IGKJ4, IGKJ3, IGKJ2, and IGKJ1. When the human V region gene is IGKV3-11, the human J region gene is 1, 2, 3, 4, or 5 of IGKJ5, IGKJ4, IGKJ3, IGKJ2, and IGKJ1. When the human V region gene is IGKV3-15, the human J region gene is 1, 2, 3, 4, or 5 of IGKJ5, IGKJ4, IGKJ3, IGKJ2, and IGKJ1. When the human V region gene is IGKV3-20, the human J region gene is 1, 2, 3, 4, or 5 of IGKJ5, IGKJ4, IGKJ3, IGKJ2, and IGKJ1. When the human V region gene is IGKV1D-39, the human J region gene is 1, 2, 3, 4, or 5 of IGKJ5, IGKJ4, IGKJ3, IGKJ2, and IGKJ1. When the human V region gene is IGKV2D-28, the human J region gene is 1, 2, 3, 4, or 5 of IGKJ5, IGKJ4, IGKJ3, IGKJ2, and IGKJ1. When the human V region gene is IGKV1D-33, the human J region gene is 1, 2, 3, 4, or 5 of IGKJ5, IGKJ4, IGKJ3, IGKJ2, and IGKJ1.

In another aspect, the non-human animal or cell as described in the present application has one or more human IGHV regions, one or more human IGHD regions, and/or one or more human IGHJ regions downstream of an encoding region of a host non-human mammalian heavy chain locus. In some embodiments, transcription directions of encoding regions of the human IGHV region, the human IGHD region, and the human IGHJ region after insertion are opposite to those of endogenous heavy chain VDJ original gene encoding regions of the host. The human IGHV region, the human IGHD region, and the human IGHJ region are operably linked together and may undergo VDJ recombination.

In some embodiments, a constant region of an animal endogenous heavy chain locus is entirely or partially modified, so that the transcription direction thereof is completely or partially opposite to that of an endogenous heavy chain variable region gene.

Therefore, the present application provides a non-human animal or cell. A genome of the non-human animal cell includes an operably linked human immunoglobulin light chain variable region gene downstream of an immunoglobulin light chain locus, and includes an operably linked human immunoglobulin heavy chain variable region gene downstream of an immunoglobulin heavy chain locus. The human immunoglobulin light chain variable region includes one human light chain V region gene and one or more (e.g., 2, 3, 4, or 5) human light chain J region genes. The human immunoglobulin heavy chain variable region includes one or more genes of human heavy chain V region, heavy chain D region, or heavy chain J region, or fragments thereof. At the same time, the genome of the non-human animal includes an endogenous immunoglobulin light chain variable region gene and a constant region gene. The endogenous immunoglobulin constant region gene is located between an endogenous immunoglobulin variable region and a human immunoglobulin variable region, and the transcription direction of the endogenous immunoglobulin variable region is opposite to those of the endogenous immunoglobulin constant region and a human immunoglobulin light chain variable region.

In one aspect, a human immunoglobulin light chain VJ region gene located downstream of the encoding region of the light chain locus of the host non-human animal includes single V region and J region of the light chain from humans arranged in reverse in a germline manner, as well as some or all intervening sequences.

In another aspect, the human immunoglobulin light chain VJ region gene located downstream of the encoding region of the light chain locus of the host non-human animal is arranged in reverse and may be operably linked to the modified host constant region, allowing for smooth VJ recombination and expression of a functional human variable region chimeric antibody.

In one aspect, a human immunoglobulin heavy chain VDJ region gene located downstream of the encoding region of the heavy chain locus of the host non-human animal includes all V regions, D regions, and J regions of the heavy chain from humans arranged in reverse in a germline manner, as well as some or all intervening sequences.

In another aspect, the human immunoglobulin heavy chain VDJ region gene located downstream of the encoding region of the heavy chain locus of the host non-human animal is arranged in reverse and may be operably linked to the modified host constant region, allowing for smooth VDJ recombination and expression of a functional human variable region chimeric antibody.

In another aspect, the human immunoglobulin genes arranged in reverse and located downstream of the encoding region of the heavy chain locus of the host non-human animal can be expressed in a constant region with a different antibody subtype from the host, allowing for smooth immunoglobulin isotype switching of B cells.

In one aspect, the inserted human immunoglobulin variable region gene DNA includes all gene encoding regions of a single functional human V region gene.

In one aspect, the inserted human immunoglobulin variable region gene DNA includes 18% to 100% of encoded reverse human light chain J region gene encoding regions, such as over 18%, over 38%, over 58%, over 78%, and all of the human light chain J region gene encoding regions.

In one aspect, the inserted human immunoglobulin variable region gene DNA includes 10% to 100% of encoded reverse human heavy chain variable (V) gene encoding region sequences, such as over 60%, over 70%, over 80%, over 90%, and all of the human heavy chain variable region gene encoding regions.

In one aspect, the inserted human immunoglobulin variable region gene DNA includes 50% to 100% of encoded reverse human heavy chain D region gene encoding regions, such as over 60%, over 70%, over 80%, over 90%, and all of the human D region gene encoding regions.

In one aspect, the inserted human immunoglobulin variable region gene DNA includes 50% to 100% of encoded reverse human heavy chain J region gene encoding regions, such as over 60%, over 70%, over 80%, over 90%, and all of the human J region gene encoding regions.

In one aspect, a length of the inserted human immunoglobulin heavy chain variable region gene DNA is approximately or at least 10 kb, 20 kb, 30 kb, 40 kb, 50 kb, 60 kb, 70 kb, 80 kb, 90 kb, 100 kb, 200 kb, 300 kb, 400 kb, 500 kb, 600 kb, 700 kb, 800 kb, 900 kb, 1000 kb, 1500 kb, 2000 kb, 2500 kb, 3000 kb, or 3500 kb.

Optionally, the non-human animal may only include the human light chain variable region gene located downstream of the encoding region of the light chain locus of the host non-human animal, or may simultaneously include the human light chain variable region gene located downstream of the encoding region of the light chain locus of the host non-human animal and the human heavy chain variable region gene located downstream of the encoding region of the heavy chain locus of the host non-human animal.

Optionally, endogenous immunoglobulin light chain variable region (V region and J region) genes of the non-human animal are not modified or deleted in any way to prevent the expression or regulation of partial host genes within the variable region from being affected. At the same time, the endogenous immunoglobulin light chain variable region is far away from the inserted human immunoglobulin light chain variable region, and an endogenous immunoglobulin light chain constant region encoding sequence is spaced therebetween, and a base length of a distance therebetween is 4 Kbp to 42 Kbp, preferably the distance is 5 Kbp. To some extent, infiltration of the endogenous variable region into the final chimeric immunoglobulin encoding gene recombination is avoided.

Optionally, endogenous immunoglobulin heavy chain variable region (V region, D region, and J region) genes of the non-human animal are not modified or deleted in any way to prevent the expression or regulation of partial host genes within the variable region from being affected. At the same time, the endogenous immunoglobulin heavy chain variable region is far away from the inserted human immunoglobulin heavy chain variable region, and an endogenous immunoglobulin heavy chain constant region encoding sequence is spaced therebetween, and a base length of a distance therebetween is 169 Kbp to 240 Kbp, preferably the distance is 202 Kbp. To some extent, infiltration of the endogenous variable region into the final chimeric immunoglobulin encoding gene recombination is avoided.

### Preparation method of non-human animals or cells

The present application discloses a method for constructing a functional human immunoglobulin variable region gene in non-human animals (e.g., mice). In the present application, the research conducted in mice is only an example. Unless otherwise specified, all non-human animals also included in the reference to mice, and mice are preferred non-human animals.

In one aspect, through site-directed recombination, the insertion of the human immunoglobulin light chain variable region gene DNA, such as human light chain κ VJ, is targeted between lgkc and Rpia genes downstream of the IgK locus on the mouse chromosome 6. In one aspect, the insertion is between coordinates 70,703,738 and 70,742,704 on the mouse chromosome 6, appropriately at a position 70,729,444, or at an equivalent position on the mouse λ locus chromosome 16. NCBI database GRCm39 is referred to for all the coordinates.

In one aspect, through site-directed recombination, the insertion of the human immunoglobulin heavy chain variable region gene DNA is targeted between Tmem121 and Igha genes downstream of the IgH locus on the mouse chromosome 12. In one aspect, the insertion is between coordinates 113,149,523 and 113,223,857 on the mouse chromosome 12, appropriately at a coordinate 113,981,732.

In some embodiments, the site-directed recombination method includes homologous recombination, excinuclease mediated recombination (e.g., CRISPR/Cas9), and site-specific recombinase mediated recombination. Combined use of one or more methods is involved.

In some embodiments, a constant region of an endogenous locus is entirely or partially modified, so that the transcription direction thereof is completely or partially opposite to that of the corresponding endogenous variable region gene. The modification involves inserting an exogenous recombination site inside or at two ends of a constant region of a non-human animal cell or animal, and then reversing the entire or part of a host constant region gene sequence through intracellular delivery of recombinases or animal breeding. The insertion may be performed through a commonly used gene editing targeting method, for example, homologous recombination or excinuclease mediated recombination (e.g., CRISPR/Cas9). The modification may also be achieved through nucleases, and related techniques are known in the prior art.

In some embodiments, a constant region of an endogenous light chain locus is entirely or partially modified, so that the transcription direction thereof is completely or partially opposite to that of the corresponding endogenous light chain variable region gene. The modification involves reverse insertion or replacement of part or entire of the constant region gene sequence of the host using a non-human animal constant region. The insertion or replacement may be performed through a commonly used gene editing targeting method, for example, homologous recombination or excinuclease mediated recombination (e.g., CRISPR/Cas9).

In some embodiments, by first inserting the modified human variable region gene downstream (the rear) of the endogenous immunoglobulin locus in the mouse cell, and then introducing a modified short exogenous recombinase binding site into the endogenous constant region of the mouse cell. The introduction of the recombinase makes the endogenous constant region of the mouse cell to partially or entirely reverse. After the modified cell is obtained by screening and identification, the cell is injected into an early embryo to prepare a chimeric animal (e.g., a mouse). Subsequent breeding may be performed to obtain an animal including a complete humanized immunoglobulin locus.

In some embodiments, by first introducing the modified short exogenous recombinase binding site into the endogenous constant region of the mouse cell, the introduction of the recombinase makes the endogenous constant region of the mouse cell to partially or entirely reverse. Then, the modified human variable region gene is inserted downstream (the rear) of the endogenous immunoglobulin locus in the mouse cell. After the modified cell is obtained by screening and identification, the cell is injected into an early animal embryo to prepare a chimeric animal (e.g., a mouse). Subsequent breeding may be performed to obtain an animal including a complete humanized immunoglobulin locus.

Optionally, in some embodiments, only the human light chain variable region gene may be introduced downstream of the encoding region of the light chain locus of the host non-human animal, or the human light chain variable region gene may be introduced downstream of the encoding region of the light chain locus of the host non-human animal while the human heavy chain variable region gene may be introduced downstream of the encoding region of the heavy chain locus of the host non-human animal.

### Method for preparing common light chain antibodies using non-human animals

Non-human animals may be used for producing humanized or chimeric antibodies specifically binding to targets. By using a standard technique and using a target (e.g., a protein or a fragment thereof) as an immunogen, antibody production may be induced in a non-human animal. For example, multiple injections of an antigen peptide or a protein (which may be combined with an adjuvant) may induce production of a polyclonal antibody; and subsequently, an immobilized antigen and techniques such as ELISA are used for monitoring the antibody titer and perform purification.

To obtain a monoclonal antibody, cells may be extracted from an immune animal and screened for a cell that produces the target antibody by a hybridoma technique. A single cell screening technique may also be used for directly analyzing an antibody sequence using DNA barcoding and single-cell sequencing without the need for hybridoma fusion.

In a modified mouse model, B cells including human light chain and/or heavy chain gene sequences are produced through genetic engineering. These modifications may include integrating the human immunoglobulin variable region and constant region genes into a mouse locus. The finally produced antibody may be entirely contributed by the human V, D, and J genes, and may be further humanized or optimized to enhance binding affinity, alter glycosylation characteristics, and the like.

By using a nucleic acid sequencing or peptide synthesis technique, an antigen binding site of the antibody may be modified to optimize the functionality thereof. All antibodies may be derived from mammals, including human and humanized antibodies. Subsequent modifications of the antibodies may also include covalent modifications or other post-translational modifications to enhance specific characteristics or functions.

### Method for preparing bispecific antibodies using common light chains

The present disclosure provides a non-human animal of which an expressed light chain has a limited lineage and may be associated with multiple heavy chains. In some embodiments, the non-human animal includes a human light chain variable region locus, and the locus has one IGK V region gene and a small number of (e.g. 1 to 5) IGK J region genes, and is operably linked downstream of an endogenous constant region gene. An endogenous constant region may be partially or entirely modified (e.g., inverted), so that the transcription direction thereof is opposite to that of an endogenous variable region gene.

The animal may be immunized with different immunogens to produce B cells expressing specific antibodies. For example, a heavy chain variable region may be cloned from the B cells and fused with a human heavy chain constant region; and a light chain is fused with a human light chain constant region for cell transfection and antibody expression.

In addition, the method may also be used for preparing a bispecific antibody. By modifying the interface of an antibody molecule (e.g., a CH3 domain), the proportion of heterodimers recovered from a recombinant cell culture may be maximized to achieve efficient production of bispecific antibodies.

Without being bound by any theory, the following examples are only for illustrating the non-human animal, preparation method and use of the present application, and not intended to limit the scope of the present application.

### Examples

### Example 1: Overall strategy

A mouse model of the present application may be established by inserting a sequence including only one functional V region and one or more J regions of a human immunoglobulin light chain variable region downstream of a mouse immunoglobulin light chain encoding locus, or by simultaneously inserting a heavy chain gene including all human-derived V, D, and J regions downstream of a mouse immunoglobulin heavy chain encoding locus. Such insertion is achieved through gene targeting in an ES cell using a well-known technique in the art. By recombinant engineering, a targeting vector including a human antibody variable region gene sequence is constructed in vitro, and the targeting vector is inserted downstream of the mouse-derived immunoglobulin locus to achieve modification of the human-derived antibody variable region gene. Due to the large size of the human immunoglobulin variable region gene, to ensure successful, effective, and accurate acquisition of a mouse including all human immunoglobulin variable regions, the present application uses a series of solutions to improve the success probability of the work. Some optional solutions include the following.

A human IGK V/J sequence selected to be inserted downstream of the mouse immunoglobulin light chain encoding locus in the present application includes only one functional human immunoglobulin light chain variable region V region, and the number of subsequent human immunoglobulin light chain variable region J regions is not limited. As described in Example 4, an optional solution of the present application is to prepare a sequence including only one human IGK V region and all human IGK J regions, and insert the sequence downstream of a mouse IgK constant region to obtain a mouse. An optional solution of the present application is to prepare a sequence including only one human IGK V region and only one human IGK J region, and insert the sequence downstream of the mouse IgK constant region to obtain a mouse. The included only one human IGK V region is linked to one or more subsequent IGK J regions in sequence, without including an intron sequence therebetween or further without including a recombination *signal sequence* (RSS) within the VJ intron. The inserted human IGK V region and IGK J regions may be rearranged or only retain a limited rearrangeable J.

In the present application, the human-derived gene is inserted into the Junk sequence downstream of the mouse-derived immunoglobulin locus to avoid affecting mouse-derived gene expression in the final target cell or mouse, as described in Example 2 below.

When ES cell targeting is performed, not all targeting vectors can be completely integrated in practical operations. ES cells are screened through high-density gene identification to obtain cells with complete gene insertion. Such identification may be performed through high-density PCR or Q-PCR. The complete integration of the targeting vectors is ultimately confirmed through high-density PCR analysis, and the probability of obtaining completely integrated ES cells during practical operations is around 10%. In Example 3, Table 6 represents a partially integral array PCR design of the first vector (vector1) during a heavy chain targeting process.

In addition, due to instability of the karyotype of mouse ES in vitro culture (Gaztelumendi N, Nogués C. Chromosome Instability in mouse Embryonic Stem Cells. Scientific Reports, Springer Science and Business Media LLC, 2014, 4(1).), only a few ES cells obtained with complete gene insertion retain chromosome integrity and germline transmission capacity after long-time in vitro culture and continuous targeting operation. In operation, a detailed karyotype analysis solution may be adopted to identify the screened ES cells, for example, karyotype examination based on general microscopy (photomicrographic), or karyotype detection based on specially designed Q-PCR (D'Hulst C, Parvanova I, Tomoiaga D, et al. Fast Quantitative Real-Time PCR-Based Screening for Common Chromosomal Aneuploidies in Mouse Embryonic Stem Cells[J]. Stem Cell Reports, Elsevier BV, 2013, 1(4): 350-359.), or based on multiple chromosomes (Barrett M T, Scheffer A, Ben-Dor A, et al. Comparative genomic hybridization using oligonucleotide microarrays and total genomic DNA. Proceedings of the National Academy of Sciences, Proceedings of the National Academy of Sciences, 2004, 101(51): 17765-17770.). However, these solutions often only reflect partial capacity of the obtained ES, and cannot represent the germline transmission capacity and the production capacity of human variable region chimeric antibodies.

In view of the above, in one feasible operation solution of the present application, an intermediate animal is produced and validated in a timely or timed manner (e.g., targeting modification of cells every 3 times), resulting in an effect that almost every modified ES cell inserted into the obtained partial human variable region may be injected into a mouse embryo to produce a chimeric mouse for validating the germline transmission capacity thereof. At the same time, with the presence of the human variable region genes operably inserted downstream of the mouse-derived immunoglobulin gene loci, the chimeric mice obtained can use the inserted human variable region genes to perform VDJ or VJ recombination and produce functional human variable region chimeric antibodies. The data serve as check points for producing final non-human animals with complete human variable regions. The reliability and functionality of the obtained process ES cells are determined from the perspectives of animal production, antibody production, and diversity of variable region domains, so as to make timely predictions of success or failure and route correction for production of a complete human variable region route. These mice carrying partial human variable regions may also be discovered with antibodies for the production of human variable region chimeric antibodies. At the same time, these mice may be used for a universal solution for producing animals with human immunoglobulin light chain variable regions, which can be used for producing a series of animals including different single human light chain variable region V regions, and can also be used for producing animals including all or part of the human light chain variable region V regions.

### Example 2: Modification of mouse immunoglobulin loci

An ES cell line was obtained from a BALB/c mouse through autonomous isolation to serve as a basic material for subsequent targeting.

Using a well-known ES targeting technique in the art, a vector element 1 (hereinafter referred to as Cpoint, an element used for introducing human variable region insertion) was knocked into a downstream location (a region between Igkc and Rpia genes, specifically IGK chr6:70,706,267. NCBI database GRCm39) of a mouse-derived IGK locus in an ES cell. After the ES cell line with the element 1 (Cpoint) knocked-in correctly was obtained, a vector element 2 (hereinafter referred to as LD, used for cooperation with Cpoint) was knocked into an upstream location (a region between an Igk J5 exon and an Igkc gene, specifically IGK chr6: 70,701,630. NCBI database GRCm39) of a mouse-derived IGK constant region using an ES targeting technique.

The solution may also be used for modification near the immunoglobulin heavy chain locus, namely, a vector element 1 (referred to as Cpoint, an element used for introducing human variable region insertion) was knocked into a downstream location (a region between an Igha gene and a Tmem121 gene, specifically IGH chr12:113,190,256. NCBI database GRCm39) of a mouse-derived IGH locus in an ES cell. Similarly, after the ES cell line with the element 1 (Cpoint) knocked-in correctly was obtained, a vector element 2 (LD, used for cooperation with the Cpoint) was knocked into an upstream location (a region between an IgH J4 exon and a Cµ locus, specifically IGH chr12: 113,391,844.) of a mouse-derived IGH constant region using an ES targeting technique.

The above positive cell line was transfected with a Cre expression plasmid, and inversion of a mouse-derived C region was achieved through a Cre/loxP recombination mechanism (Zheng, B et al. "Engineering a mouse balancer chromosome." Nature genetics vol. 22,4 (1999): 375-8. doi:10.1038/11949). The above positive cell line was further transfected with a transposase expression plasmid, and a screening marker fragment was deleted through a transposition mechanism (Transposase/Transposon system) (Maragathavally, K. J., et al. "Chimeric Mos1 and piggyBac transposases result in site-directed integration." The FASEB journal 20.11 (2006): 1880-1882. Wilson, Matthew H., Craig J. Coates, and Alfred L. George. "PiggyBac transposon-mediated gene transfer in human cells." Molecular therapy 15.1 (2007): 139-145.).

By the recombinase mediated cassette exchange technique (RMCE) (Wallace, Helen A C et al. "Manipulating the mouse genome to engineer precise functional syntenic replacements with human sequence." Cell vol. 128,1 (2007): 197-209. doi:10.1016/j.cell.2006.11.044, Prosser, Haydn M et al. "Mosaic complementation demonstrates a regulatory role for myosin VIIa in actin dynamics of stereocilia." Molecular and cellular biology vol. 28,5 (2008): 1702-12. doi:10.1128/MCB.01282-07), vector targeting was performed once or in batches. After each time of vector insertion, the screening marker fragment was deleted through the transposition mechanism until the last vector was inserted, resulting in the ES cell line with the target human-derived antibody variable region encoding gene (VDJ) completely inserted.

The ES cells subjected to quality control by PCR and karyotype Q-PCR array analysis were injected into mouse blastocyst cavities according to a method in "Manipulating the Mouse Embryo: A Laboratory Manual, Fourth Edition (Cold Spring Harbor Laboratory Press, 2014)" to obtain chimeric mice. The chimeric mice were bred with BALB/c mice, and after genotype identification and functional validation, mice including human immunoglobulin light chain variable region genes and including or not including human immunoglobulin heavy chain variable region genes were finally obtained. To obtain functional validation data more quickly, an optional solution was to inject the ES cells into mouse blastocysts that could not undergo functional VDJ rearrangement (e.g., Rag1 gene deficient mice), and the obtained chimeric mice could be directly validated for immune system function without breeding.

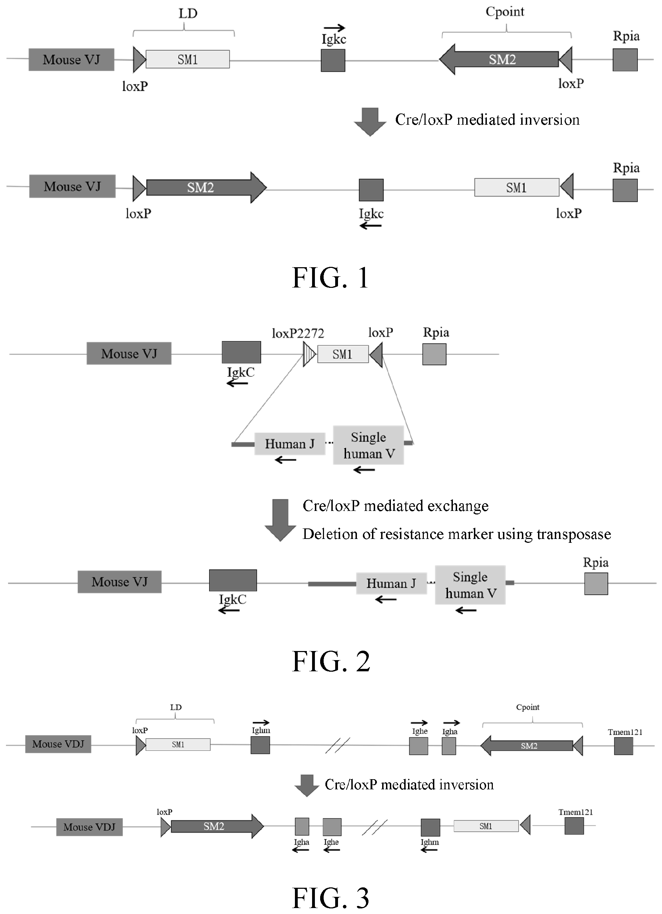

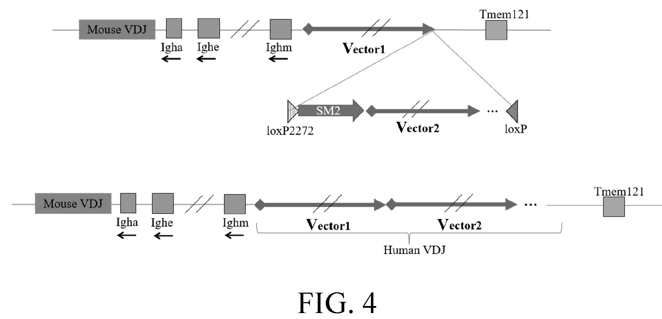

A preparation diagram and the vector design are shown in FIG. 1 to FIG. 4, FIG. 1 shows an IgK constant region Cpoint and LD targeting, as well as C-region modification; FIG. 2 shows a schematic diagram of inserting a light chain human variable region, illustrating IGK-vector targeting and resistance marker deletion; FIG. 3 shows an IgH constant region Cpoint and LD targeting, as well as C-region modification; and FIG. 4 shows a schematic diagram of inserting a heavy chain human variable region, illustrating IGH-vector targeting and resistance marker deletion.

### Example 3: Selection of light chain VJ regions: non-rearranged VJ and rearranged VJ

### 3.1 Construction of Cpoint targeting vector

A targeting vector was composed of homologous arms at two ends (sequences on two sides of an insertion site), a screening marker (SM for short) 2, loxP, and a transposon PB fragment, and as shown in FIG. 1 and FIG. 3, these fragments were enzymatically linked to a commercial vector PMD18T to construct the Cpoint targeting vector. As described in Example 2, the solution of the present application may be used not only for modification near the immunoglobulin light chain locus, but also for modification near the immunoglobulin heavy chain locus.

### (1) Cpoint modification near the light chain

By using the well-known ES targeting technique in the art, the Cpoint targeting vector was knocked into a downstream location of the mouse IgK constant region locus in an ES cell.

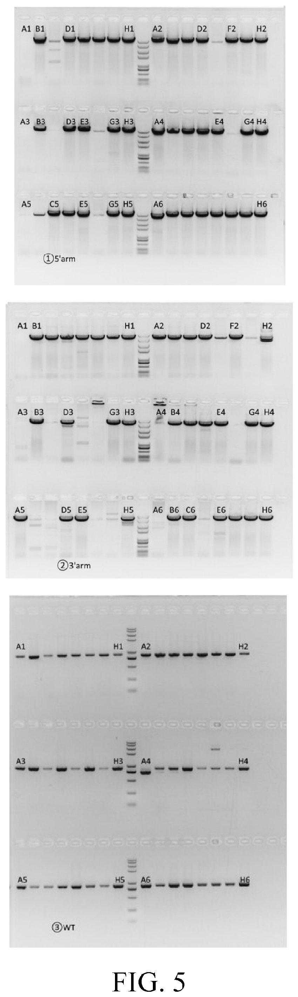

By PCR and sequencing identification, it was confirmed that the IgK-Cpoints of clones C6, E5, G4, D2, and A2 were correctly targeted. The Cpoint identification solution is as shown in Table 1, and the PCR identification results are shown in FIG. 5.

**Table 1 Cpoint identification solution for light chains**

| **Serial No.** | **Primer name** | **Primer sequence** | **SEQ ID NO** | **Product size** |
|---|---|---|---|---|
| (1) 5arm | Igk-Cp-5tF1 | ATCCAAAGAAGGCCATTCAATTCTAGCACAC | 1 | KI=6978 bp |
| | Igk-Cp-5tR1 | CTTGGGGTCTCTTCCTTCATGCATCGAA | 2 | |
| | | | | WT=/bp |
| (2) 3arm | Igk-Cp-3tF1 | AAGTACTCGCCGATAGTGGAAACCGAC | 3 | KI=5643 bp |
| | Igk-Cp-3tR1 | ATGTCAGCCTTCCCCACTTCATTATTACCTC | 4 | |
| | | | | WT=/bp |
| (3) WT | Igk-Cp-wtF1 | ATCCGTGTTGAGTTTCCACAAGTACTGTC | 5 | WT=865 bp |
| | Igk-Cp-wtR1 | ATGACTTTTGCTGGCTGTAGATTTTACCTC | 6 | |
| | | | | KI=/bp |

### (2) Cpoint modification near the heavy chain

By using the well-known ES targeting technique in the art, the Cpoint targeting vector was knocked into a downstream location of the mouse IGH constant region locus in an ES cell.

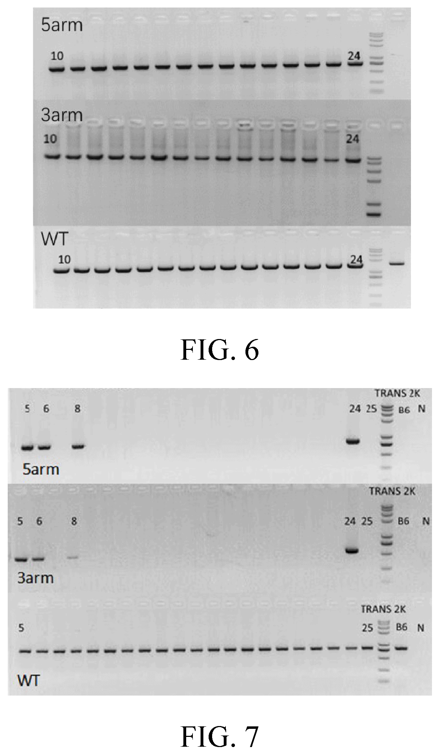

By PCR identification, it was confirmed that the IGH-Cpoints of clones 10 to 24# were correctly targeted. The Cpoint identification solution is as shown in Table 2, and the PCR identification results are shown in FIG. 6.

**Table 2 Cpoint identification solution for heavy chains**

| **Serial No.** | **Primer name** | **Primer sequence** | **SEQ ID NO** | **Product size** |
|---|---|---|---|---|
| (1) 5arm | Igh-Cp-5tF1 | actccgaggcacatgcttag | 7 | KI=756 bp |
| | Igh-Cp-5tR1 | TCCTACATCGAAGCTGAAAGCACGAG | 8 | WT=/bp |
| (2) 3arm | Igh-Cp-3tF1 | TACACATTTCTTCTCAAGCACTGGCTAT | 9 | KI=5805 bp |
| | Igh-Cp-3tR1 | cttcgtctcttctgttatttggcgactcc | 10 | WT=/bp |
| (3) WT | Igh-Cp-wtF1 | ctgtgtagacctcaaagtcagcca | 11 | WT=1561 bp |
| | Igh-Cp-wtR1 | acctccaacagttatcctgcgttaa | 12 | |
| | | | | KI=/bp |

### 3.2 Construction of LD targeting vector

A targeting vector was composed of homologous arms at two ends (sequences on two sides of an insertion site), a screening marker (SM for short) 1, loxP, and a transposon PB fragment, and as shown in the figures, these fragments were enzymatically linked to a commercial vector PMD18T to construct the LD targeting vector. As described in Example 2, the solution of the present application may be used not only for modification near the immunoglobulin light chain locus, but also for modification near the immunoglobulin heavy chain locus.

### (1) Cpoint modification near the light chain

For the ES cells correctly targeted with the Cpoint, the LD targeting fragment was knocked into an upstream location (a region between an Igk J5 exon and an Igkc gene, specifically IGK chr6: 70,701,630. NCBI database GRCm39) of a mouse-derived IgK constant region using an ES targeting technique.

By PCR identification, it was confirmed that the IGK-LDs of clones 5, 6, 8, and 24# were correctly targeted. The LD identification solution is as shown in Table 3, and the PCR identification results are shown in FIG. 7.

**Table 3 LD identification solution for light chains**

| **Serial No.** | **Primer name** | **Primer sequence** | **SEQ ID NO** | **Product size** |
|---|---|---|---|---|
| (1) 5arm | Igk-LD-5tF1 | ACTTCTACACAGCCATCGGT | 13 | KI=842 bp |
| | Igk-LD-5tR1 | CGTGGATATGTCCTGCGGGT | 14 | WT=/bp |
| (2) 3arm | Igk-LD-3tF1 | AAGAAGATGTTGGCGACCTCGTAT | 15 | KI=572 bp |
| | Igk-LD-3tR1 | CGTGGATATGTCCTGCGGGT | 14 | WT=/bp |
| (3) WT | Igk-LD-wtF1 | CAGAGGATGAAACAGCGCAGAT | 16 | WT=791 bp |
| | Igk-LD-wtR1 | TGCATTTTATTTCTGAAATCTCCAAT | 17 | KI=/bp |

### (2) Cpoint modification near the heavy chain

For the ES cells correctly targeted with the Cpoint, the LD targeting fragment was knocked into an upstream location (a region between an IgH J4 exon and a Cµ locus, specifically IGH chr12: 113,391,844. NCBI database GRCm39) of a mouse-derived IgH constant region using an ES targeting technique.

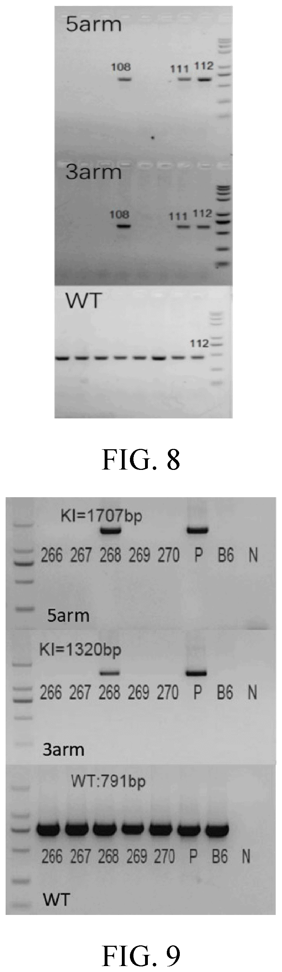

By PCR identification, it was confirmed that the IGH-LDs of clones 108#, 111#, and 112# were correctly targeted. The LD identification solution is as shown in Table 4, and the PCR identification results are shown in FIG. 8.

**Table 4 LD identification solution for heavy chains**

| **Serial No.** | **Primer name** | **Primer sequence** | **SEQ ID NO** | **Product size** |
|---|---|---|---|---|
| (1) 5arm | Igh-LD-5tF1 | AGGTAAGAATGGCCTCTCCAGG | 18 | KI=642 bp |
| | Igh-LD-5tR1 | AAGAACCAGCCGGATCTGCAA | 19 | WT=/bp |
| (2) 3arm | Igh-LD-3tF1 | CCTACCGGTGGATGTGGAAT | 20 | KI=605 bp |
| | Igh-LD-3tR1 | GTGCGGAACATTCCTCACAAATC | 21 | WT=/bp |
| (3) WT | Igh-LD-wtF1 | AGGTAAGAATGGCCTCTCCAGG | 18 | WT=444 bp |
| | Igh-LD-wtR1 | GTGCGGAACATTCCTCACAAATC | 21 | KI=/bp |

### 3.3 Construction of Cre expression vector

A Cre expression plasmid was composed of a eukaryotic promoter, a Cre encoding gene, and a terminator, and these fragments were enzymatically linked to a commercial vector PMD18T to construct the Cre expression vector.

The Cpoint and LD correctly targeted ES cells were transfected with the Cre expression vector to achieve inversion of the mouse-derived C region through a Cre/loxP recombination mechanism.

### (1) Inversion modification of light chain constant region

By PCR identification, it was confirmed that the light chain constant region of clone 268# was inverted correctly. An identification solution for inversion of the light chain constant region is shown in Table 5, and the identification results are shown in FIG. 9.

**Table 5 Identification solution for inversion of light chain constant region**

| **Serial No.** | **Primer name** | **Primer sequence** | **SEQ ID NO** | **Product size** |
|---|---|---|---|---|
| (1) 5arm | K-INV-5TR1 | CTTCATCACATCTCGAGCAAGACGTT | 22 | KI=1707 bp |
| | K-INV-5TF1 | ACTTGCTATTTGAACATAAACTAGGCCAAC | 23 | |
| | | | | WT=/bp |
| (2) 3arm | K-INV-3TR1 | TGCGCAGCTGTGCTCGACGT | 24 | KI=1320 bp |
| | K-INV-3TF1 | TGGCCTCAGAACTTGGTGTCACTCA | 25 | |
| | | | | WT=/bp |
| (3) WT | K-INV-wtF1 | CAGAGGATGAAACAGCGCAGAT | 16 | WT=791 bp |
| | K-INV-wtR1 | TGCATTTTATTTCTGAAATCTCCAAT | 17 | |
| | | | | KI=/bp |

### (2) Inversion modification of heavy chain constant region

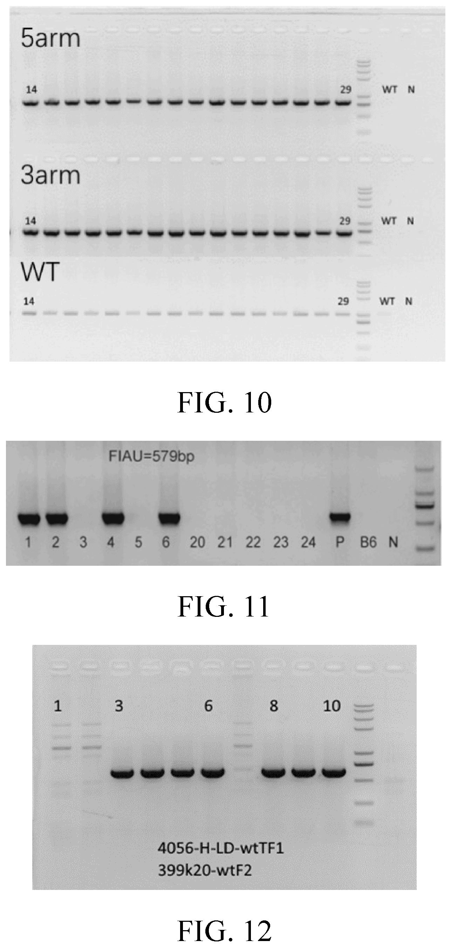

By PCR identification, it was confirmed that the heavy chain constant regions of clones 14 to 29# were correctly inverted. An identification solution for inversion of the heavy chain constant region is shown in Table 6, and the identification results are shown in FIG. 10.

**Table 6 Identification solution for inversion of heavy chain constant region**

| **Serial No.** | **Primer name** | **Primer sequence** | **SEQ ID NO** | **Product size** |
|---|---|---|---|---|
| (1) 5arm | H-INV-5TR1 | ATTCAATAAACTCTTTCCCCTTGACC | 26 | KI=821 bp |
| | H-INV-5TF1 | TGCTATTTGAACATAAACTAGGCCAAC | 27 | WT=/bp |
| (2) 3arm | H-INV-3TR1 | TAGTTGCCAGCCATCTGTTGTTTGC | 28 | KI=723 bp |
| | H-INV-3TF1 | tgatgtGAgcaccttggctaccattgcttatg | 29 | WT=/bp |
| (3) WT | H-INV-wtF1 | cataaacgcacaaacatacagatatatgct | 30 | WT=955 bp |
| | H-INV-wtR1 | taaatggcatgatgtacgcacctt | 31 | |
| | | | | KI=/bp |

### 3.4 Construction of transposase expression vector

A transposase expression plasmid was composed of a eukaryotic promoter, a transposase encoding gene, and a terminator, and these fragments were enzymatically linked to a commercial vector PMD18T to construct the transposase expression vector.

The inverted positive cell line was further transfected with a transposase expression plasmid, and a screening marker fragment was deleted through a transposition mechanism.

By PCR identification, it was confirmed that the light chain resistance markers of clones 1, 2, 4, and 6# were deleted correctly. An identification solution for deletion of the light chain resistance markers is shown in Table 7, and the identification results are shown in FIG. 11.

**Table 7 Identification solution for deletion of light chain resistance markers**

| **Serial No.** | **Primer name** | **Primer sequence** | **SEQ ID NO** | **Product size** |
|---|---|---|---|---|
| (1) FIAU | K-FIAU-tF1 | CAGAGGATGAAACAGCGCAGAT | 16 | FIAU=579 bp |
| | K-FIAU-tR1 | GGGCACATATTAATTATCCAGCACTAGGAG | 32 | |

By PCR identification, it was confirmed that the heavy chain resistance markers of clones 3 to 6# and 8 to 10# were correctly deleted. An identification solution for deletion of the heavy chain resistance markers is shown in Table 8, and the identification results are shown in FIG. 12.

**Table 8 Identification solution for deletion of heavy chain resistance markers**

| **Serial No.** | **Primer name** | **Primer sequence** | **SEQ ID NO** | **Product size** |
|---|---|---|---|---|
| (1) FIAU | H-FIAU-tF1 | AGGTAAGAATGGCCTCTCCAGG | 18 | FIAU=644 bp |
| | H-FIAU-tR1 | cactcacacataaaagttgacactggcact | 33 | |

### 3.5 Construction of IGK VJ targeting vector

As shown in FIG. 2, the IGK VJ targeting vector was constructed using a Red/ET recombination technique (Rivero-Müller, Adolfo et al. "Assisted large fragment insertion by Red/ET-recombination (ALFIRE)--an alternative and enhanced method for large fragment recombineering." Nucleic acids research vol. 35,10 (2007): e78. doi:10.1093/nar/gkm250). The IGK VJ targeting vector was composed of a human IGK V/J sequence, homologous arms at two ends (sequences on two sides of an insertion site), and carried a screening marker (SM for short) 2 and a transposon PB fragment for homologous recombination screening and transposon resistance deletion.

As described in Example 1, the human IGK V/J sequence included only one functional human immunoglobulin light chain variable region V region and one or more human immunoglobulin light chain variable region J region sequences. The selected only one human IGK V region was linked to one or more subsequent IGK J regions in sequence, without including an intron sequence therebetween or further without including a recombination signal sequence (RSS) within the VJ intron.

The directly linked IGKV1-5 and IGKJ1 sequences did not include a recombination signal sequence (RSS) therebetween. The sequences were inserted downstream of the mouse IgK constant region, and the obtained mouse was immunized and could be used for screening for an antibody molecule with a light chain IGKV1-5/J1, having a Sequence 1.

The directly linked IGKV1-39, IGKJ1, IGKJ2, and IGKJ5 sequences included a recombination signal sequence (RSS) therebetween. The sequences were inserted downstream of the mouse IgK constant region, and the obtained mouse was immunized and could be used for screening for an antibody molecule with light chains IGKV1-39/J1, IGKV1-39/J2, and IGKV1-39/J5, having a Sequence 2.

The present application adopted the well-known ES targeting technique in the art, and the IGK VJ targeting vector was knocked into a downstream location, which could be the same as the Cpoint knock-in location, of the mouse IgK locus in an ES cell. The targeting technique used herein was homologous recombination, but other site-directed recombination methods could also be used, including excinuclease mediated recombination (e.g., CRISPR/Cas9) and site-specific recombinase mediated recombination.

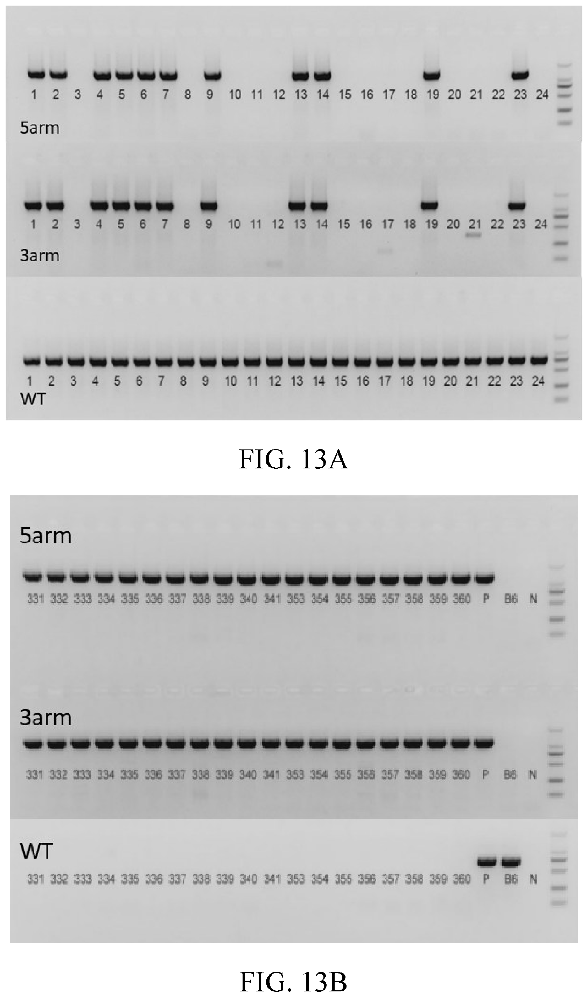

By PCR identification, it was confirmed that the light chains IGKV1-39/J1-J2-J5 of clones 1, 2, 4 to 7, 9, 13, 14, 19, and 23# were correctly inserted. An identification solution for insertion of the light chain IGK VJ is shown in Table 9, and the identification results are shown in FIG. 13A.

**Table 9 Identification solution for insertion of light chain IGK VJ**

| **Serial No.** | **Primer name** | **Primer sequence** | **SEQ ID NO** | **Product size** |
|---|---|---|---|---|
| (1) 5arm | K-5TR1 | CCGTAGCCAGCTTTCCTGAT | 34 | KI=1314 bp |
| | K-5TF1 | CAAACTCGAGAATTGATTGCACATTTT | 35 | WT=/bp |
| (2) 3arm | K-3TR1 | TCTAGAGCCGCTGTTGTGAC | 36 | KI=1436 bp |
| | K-3TF1 | GATCTCCAGCACCACATTAAACCC | 37 | WT=/bp |
| (3) WT | K-wtF1 | CAGAGGATGAAACAGCGCAGAT | 16 | WT=791 bp |
| | K-wtR1 | TGCATTTTATTTCTGAAATCTCCAAT | 17 | KI=/bp |

By PCR identification, it was confirmed that the light chains IGKV1-5/J1 of clones 331 to 360# were correctly inserted. An identification solution for insertion of the light chain IGK VJ is shown in Table 10, and the identification results are shown in FIG. 13B.

**Table 10 Identification solution for insertion of light chain IGK VJ**

| **Serial No.** | **Primer name** | **Primer sequence** | **SEQ ID NO** | **Product size** |
|---|---|---|---|---|
| (1) 5arm | K-5TR1 | AGATCCTCCTTGTGACAGCTC | 38 | KI=1439 bp |
| | K-5TF1 | GCAAAGGTTCTATAAAGCTGAGAGAC | 39 | WT=/bp |
| (2) 3arm | K-3TR1 | TCAGTGCATGGGGACATCATC | 40 | KI=1304 bp |
| | K-3TF1 | GATCTCCAGCACCACATTAAACCC | 37 | WT=/bp |
| (3) WT | K-wtF1 | CAGAGGATGAAACAGCGCAGAT | 16 | WT=791 bp |
| | K-wtR1 | TGCATTTTATTTCTGAAATCTCCAAT | 17 | KI=/bp |

where
the sequence of IGKV1-5/J1 is as follows: 
Tgcagctgtgcccagcctgccccatcccctgctcatttgcatgttcccagagcacaacctcctgccctgaagccttattaataggctgg
tcacactttgtgcaggagtcagacccagtcaggacacagcatggacatgagggtccccgctcagctcctggggctcctgctgctctggctc
ccaggtaaggaaggagaacactaggaatttactcagcccagtgtgctcagtactgcctggttattcagggaagtcttcctataatatgatcaat
agtatgaatatttgtgtttctatttccaatctcaggtgccaaatgtgacatccagatgacccagtctccttccaccctgtctgcatctgtaggagac
agagtcaccatcacttgccgggccagtcagagtattagtagctggttggcctggtatcagcagaaaccagggaaagcccctaagctcctga
tctataaggcgtctagtttagaaagtggggtcccatcaaggttcagcggcagtggatctgggacagaattcactctcaccatcagcagcctg
cagcctgatgattttgcaacttattactgccaacagtataatagttattctccgtggacgttcggccaagggaccaaggtggaaatcaaacgtg
agtagaatttaaactttgcttcctcagttgtctgtgtcttctgttccctgtgtctatgaagtgatctataaggtgactctgcaatcagcctctgatatc
cttcagggaaaagataaagataagtctgtagtcaaactcgagaattgattgcacattttctttgaagagcaagcaagat (SEQ ID
NO:41)
the sequence of IGKV1-39/J1-J2-J5 is as follows:

cacatgaaacaatgggaaccatgtgacaatcacagaggtgttgttactatagcaaaagggattgttactctccacatccctttaagtaac
ttgaaggcctgatagacccaccctctaagacttcattagacattccctacgaatggttatactctcctgtatactcccaatacaactctaaaatat
attattccatatagtccttaggtttgtattaaagtttgactttttccttcaaaatatctcttgtcacaacagcggctctagagagaaatacattccctc
caggcaaatctatgctgcgctggtctgacctgggaccctggggacattgcccctgtgctgagttactaagatgagccagccctgcagctgtg
ctcagcctgccccatgccctgctgattgatttgcatgttccagagcacagcctcctgccctgaagactttttatgggctggtcgcaccctgtgc
aggagtcagtctcagtcaggacacagcatggacatgagggtccccgctcagctcctggggctcctgctactctggctccgaggtaaggat
ggagaacactaggaatttactcagccagtgtgctcagtactgactggaacttcagggaagttctctgataacatgattaatagtaagaatatttg
tttttatgtttccaatctcaggtgccagatgtgacatccagatgacccagtctccatcctccctgtctgcatctgtaggagacagagtcaccatca
cttgccgggcaagtcagagcattagcagctatttaaattggtatcagcagaaaccagggaaagcccctaagctcctgatctatgctgcatcca

gtttgcaaagtggggtcccatcaaggttcagtggcagtggatctgggacagatttcactctcaccatcagcagtctgcaacctgaagattttgc
aacttactactgtcaacagagttacagtacccctcccacagtgttacaagtcataacataaacctccaaggaagcagatgtgtgaggacgag
ccacccccagatgctcctcctggtgcctccatctgctgagagcatttctcaaactcagtcaggttttgaaagtcattgggagacttttgtagaggg
gaccagggaggctcctctgaactctaagcctcttttgcccctatccccaggagaaaagatgtgacaatgcctgtcctgattgaataaggaag
agatacaagtccacctgaggagtctgtgttatgggataattggaatttacacagcaaaagagaagctattctcggtatttcaaggagaaattgtt
caagttgaataaattagagtctaaactagtctttttgaagcctacggtatgttattcgtgaagcagccactagagacaggggattctcaggtgct
cctgcagaagtcagagtgcacctgcccctggtggtatgtgctgagtaccgtgtgatgatcctcagaattcaggtcacctgctcagggctaatc
tgagagaaggtctctcttcagttgaattttgaaagacaattagcagttcacaagctaacccaggtggacaaagatgttcccaagcagaggga
gtgcttgtgaaagctggaggccatagaaaaactctaaggagtgtagggaggtgggagtaatgtatggaaggggtggagatggaaggttaa
gagagatacaaggctgcaaaaatggagctggactcaaaagaaaatactgaaaaggtcttcagtgttgttgatgagattactatggaaacact
atggaacactgggactccatggcagctccaaagatggcatgcgcctggtccagctcagtaagagctgagctcttcctgtgctgtgaaaaca
gacaaaccaaccaagtaaagtctacttttctactctattagtcttcactttggtttcgtataccatctggagctacatttcaaaatgcatttcaaagtt
atgagccttaagttgatatatatttagtctaccttttttaaataacattgcagcaaaggagaagataaaatagtaagacaacctgtaattattactc
attgagaagctgatgatttccataattacactaaatgaagtttatcctttgcaaaagcccccccagcccaccccaaaagaaagtacaaaaaaa
ctggccatttttttaattgcttgtttttctttgtaattaacattcagtctactttctaaaaaataaataaataataagcagtccagatgtggcaagttgc
taaagaaaggaaccatcaggccatagacgtaaatatattctcttcttggattttaggtctcacctaagaaaataaacacatgctatgtcagagaa
gcctcagggcttccacacctgctcgaaaagggagttgagcttcagcagctgacccaggactctgttcccctttggtgagaagggtttttgttc
agcaagacaatggagagctctcactgtggtggacgttcggccaagggaccaaggtggaaatcaaacgtgagtagaatttaaactttgcttcc
tcagttgtctgtgtcttctgttccctgtgtctatgaagtgatctataaggtgactctgcaatcagcctctgatatccttcagggaaaagataaagat
aagtctgtagtcaaactcgagaattgattgcacatttctttgaagagcaagcaagattcagtcattgggtgagaataacttgtctaagtaatagc
ttcagaaatgtcctggggaacataacatgttctggacagagccttggtcaattgtcagaaagggagtttttgtataggagggaagttaagagg
aaccattgtgtgtgGaCttttggccaggggaccaagctggagatcaaacgtaagtactttttccactgattcttcactgttgctaattagtttac
tttgtgttcctttgtgtggattttcattagtcggatgccagggatctaacaaacttcattcccaggttaggtacagaggaggggaaattagttcatc
ttaaggaacagccaagcgctagccagttaagtgaggcatctcaattgcaagattttctctgcatcggtcaggttagtgatattaacagcgaaaa
gagatttttgttaaggggaaagtaattaagttaacactgtggTGGaccttcggccaagggacacgactggagattaaacgtaagtaattttttc
actattgtcttctgaaatttgggtctgatggccagtattgacttttagaggcttaaataggagtttggtaaagattggtaaatgagggcatttaaga
tttgccatgggttgcaaaagttaaactcagcttcaaaaatggatttggagaaaaaaagattaaattgctctaaactgaatgacacaaagtaaaa
aaaaaaagtgtaactaaaaaggaacccttgtatttctaaggagcaaaagtaaatttatttttgttcactcttgccaaatattgtattggttgttgctg
attatgcatgatacagaaaagtggaaaaatacatttttagtctttctcccttttgtttgataaattatttgtcagacaacaataaaaatcaatagca
cgccctaagaaaaatcagggaaaagtgaagtgtacctatttgctatgtagaagaggcagcttacttgaaaatcagcagcaatgttgtttttaga
gtctgtaataagtaataaactcaaaaagacacattctataggaataagggcttcacagatagagctcatttttaaaaatccaatttgtacattag
actaaacgtgaaattatctcttattgtaatggtggaaaggtggttattcccaaaagctcaatctcaaagaaatgtgtttaatgaaaaaaagtaaa
taattgcatttttaatgaccgtgggtctgtgaaaaaaataggaaatatttaaagagtatgttctttcattatcctctgttattacttgtctacatttttat
tctgccaagaaggccgtggcaccgcgagctgtagacagagccgcggtctttctcgattgagtggctttggtggccatgccaccgcgctctt
ggggcagccgccttgccgctagtggccgtggccaccctgtgtctgcccgattgatgctgccgtagccagctttcctgatgcacagtgataca

aataatgccactaagggaaagagaac (SEQ ID NO:42)

### 3.6 Construction of IGH vector1 targeting vector

As shown in FIG. 4, by the Red/ET recombination technique (Rivero-Müller, Adolfo et al. "Assisted large fragment insertion by Red/ET-recombination (ALFIRE)--an alternative and enhanced method for large fragment recombineering." Nucleic acids research vol. 35,10 (2007): e78. doi:10.1093/nar/gkm250), a transposon, a promoter, loxP, and a screening marker Neo were inserted at the 5 end of the vector, and loxP, a screening marker Puro, and a transposon element were inserted at the 3 end of the vector, to construct the IGH vector1 targeting vector.

By the recombinase mediated cassette exchange technique (RMCE), the human IGH vector1 was performed targeted.

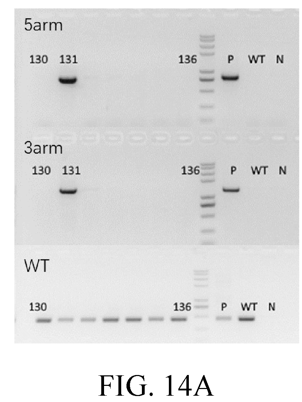

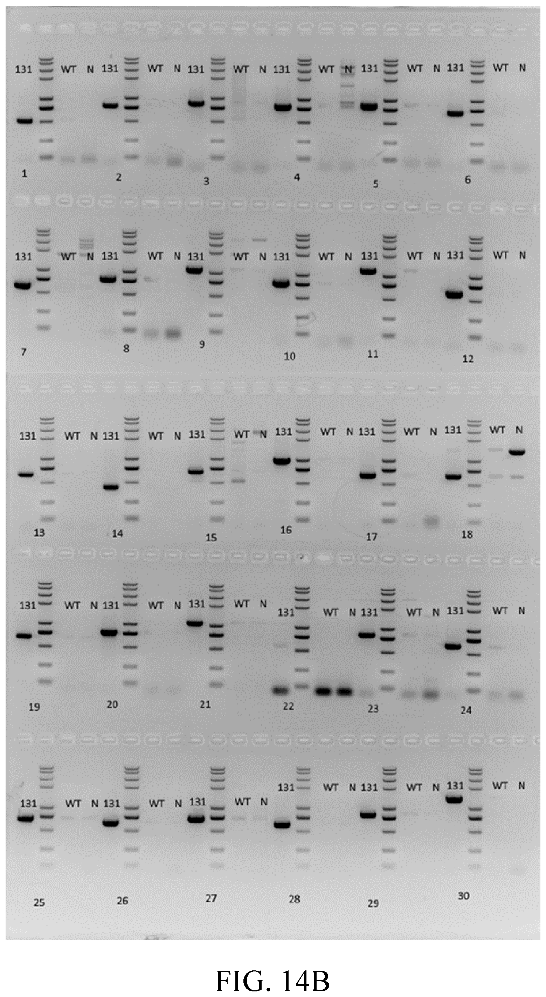

By PCR identification, it was confirmed that the IGH vector1 of clone 131# was correctly targeted. An identification solution for targeting is shown in Table 11, an identification solution for targeting integrity is shown in Table 12, the PCR identification results of the IGH vector1 targeting are shown in FIG. 14A, and the PCR identification results of the IGH vector1 targeting integrity are shown in FIG. 14B.

**Table 11 Identification solution for IGH vector1 targeting**

| **Serial No.** | **Primer name** | **Primer sequence** | **SEQ ID NO** | **Product size** |
|---|---|---|---|---|
| (1) 5arm | H-INV-5TR1 | ATTCAATAAACTCTTTCCCCTTGACC | 26 | KI=821 bp |
| | H-INV-5TF1 | TGCTATTTGAACATAAACTAGGCCAAC | 27 | WT=/bp |
| (2) 3arm | H-INV-3TR1 | TAGTTGCCAGCCATCTGTTGTTTGC | 28 | KI=723 bp |
| | H-INV-3TF1 | tgatgtGAgcaccttggctaccattgcttatg | 29 | WT=/bp |
| (3) WT | H-INV-wtF1 | cataaacgcacaaacatacagatatatgct | 30 | WT=955 bp |
| | H-INV-wtR1 | taaatggcatgatgtacgcacctt | 31 | KI=/bp |

**Table 12 Identification solution for IGH vector1 targeting integrity**

| **Serial No.** | **Detailed primer name** | **Primer sequence** | **SEQ ID NO** | **Product size (bp)** |
|---|---|---|---|---|
| 1 | H-vector-fF1 | tcttaaatccctgatcttgctcatctgtgtcg | 43 | 500 |
| | H-vector-fR1 | atgctgatgataaaaccacacagaaaataccttg | 44 | |
| 2 | H-vector-fF2 | TTAATAGGAGGCTTCCTCACAATGAAACT | 45 | 769 |
| | | TTTAGAA | | |
| | H-vector-fR2 | GTCCCCGTCCTCACCATTCAGT | 46 | |
| 3 | H-vector-fF3 | tgacctgggtttaacctgtcctggaac | 47 | 855 |
| | H-vector-fR3 | tgacactgtggttataataactccccgaac | 48 | |
| 4 | H-vector-fF4 | gcaagggaaggctcccaaacagacga | 49 | 787 |
| | H-vector-fR4 | AAGGATGTGATCAAGTCCCTGCCGCAAA | 50 | |
| 5 | H-vector-fF5 | actgaagaacatctcctagcagcaacacc | 51 | 834 |
| | H-vector-fR5 | ttcatgtttgaaggataatttcaccagctatactgttcg | 52 | |
| 6 | H-vector-fF6 | attgacttttaaaatgcagtgtagatatgtcagaga | 53 | 693 |
| | H-vector-fR6 | tcccgagtagctggaactatagatgc | 54 | |
| 7 | H-vector-fF7 | atcatgccaagcacagccgta | 55 | 600 |
| | H-vector-fR7 | ccaggagaaattccccacgtcca | 56 | |
| 8 | H-vector-fF8 | acctataaaacattaacttagaacaggggttcaattca | 57 | 763 |
| | H-vector-fR8 | ggctgattttcacagttatggaccc | 58 | |
| 9 | H-vector-fF9 | TCCCTACTGTTAAGCCACAAATCATCGTA | 59 | 1077 |
| | H-vector-fR9 | TTCCTGACCTTCTTCCCGTGTCC | 60 | |
| 10 | H-vector-fF10 | ctgtgctcagcaaagaggagaacttact | 61 | 727 |
| | H-vector-fR10 | aagacttcagagcacagactaagagc | 62 | |
| 11 | H-vector-fF11 | TGGTCTCCCATGATTCTGTTCCCAGGT | 63 | 1127 |
| | H-vector-fR11 | CAGGTGCTGAACAGATACCAGTTTCAC | 64 | |
| 12 | H-vector-fF12 | ctgtgctcagcaaagaggagaacttact | 61 | 578 |
| | H-vector-fR12 | CAGGTGCTGAACAGATACCAGTTTCAC | 64 | |
| 13 | H-vector-fF13 | tcacggagtgtctcacagtaacagtgc | 65 | 623 |
| | H-vector- | tcgagtacatttatattttgaagtcctataatcaagcctt | 66 | |
| | fR13 | | | |
| 14 | H-vector-fF14 | agtttcttattcaagccttataattgctca | 67 | 419 |
| | H-vector-fR14 | cacatcttcttttcagatcagctcgt | 68 | |
| 15 | H-vector-fF15 | ttttatctggggtctgccgtctcc | 69 | 676 |
| | H-vector-fR15 | gctcacactgacttctccttaccgta | 70 | |
| 16 | H-vector-fF16 | GCACACTAGGCATGTCCAATTCTTACCTG G | 71 | 983 |
| | H-vector-fR16 | gctcacactgacttctccttaccgta | 70 | |
| 17 | H-vector-fF17 | gctggatgtcttagaaagagctcaaggtcctt | 72 | 639 |
| | H-vector-fR17 | aaggaaactgtatacataatgaaggactagatagg | 73 | |
| 18 | H-vector-fF18 | aatattagccaatggactatgagagtgac | 74 | 627 |
| | H-vector-fR18 | taaaagatccaaatgaaaagccaaggcaac | 75 | |
| 19 | H-vector-fF19 | tcactgagatctttacttcctttatcacgtt | 76 | 646 |
| | H-vector-fR19 | attatgttttatagtatatgtaagtgcaaactggg | 77 | |
| 20 | H-vector-fF20 | gacatgctaaagaaaatttgccaaatagcg | 78 | 772 |
| | H-vector-fR20 | aactacaggacagagataaacggccta | 79 | |
| 21 | H-vector-fF21 | ggagctaaagttgacaaataaaattgtatgtatttaaggt | 80 | 1075 |
| | H-vector-fR21 | tctgtacatctttgaggtttgccggtt | 81 | |
| 22 | H-vector-fF22 | gcacatcaacaaaataaatagacagctatccag | 82 | 562 |
| | H-vector-fR22 | agatatcgctggagcatctatatctctg | 83 | |
| 23 | H-vector-fF23 | ctgtgcatccagacccacgag | 84 | 819 |
| | H-vector-fR23 | cccctaatatcctgagtgtcccgtg | 85 | |
| 24 | H-vector-fF24 | atattcctgatgaacatagacctgaaagtcct | 86 | 617 |
| | H-vector-fR24 | gttggataggattttgttcttgattatactctca | 87 | |
| 25 | H-vector-fF25 | ctgtggctactttctcatcttaaggacga | 88 | 705 |
| | H-vector-fR25 | ggtaacaacccatcacacggctt | 89 | |
| 26 | H-vector-fF26 | gcacgtttgtctccagcacctaagtt | 90 | 626 |
| | H-vector-fR26 | tcccctggtgcatgaactct | 91 | |
| 27 | H-vector-fF27 | CATTCCAGGGCTCAAGGCAT | 92 | 716 |
| | H-vector-fR27 | GTGGCATGTCCATCTGTAGCTTGTC | 93 | |
| 28 | H-vector-fF28 | TCCACTGTGTTGTGTGTAGGCTCATCTG | 94 | 601 |
| | H-vector-fR28 | GTGGCATGTCCATCTGTAGCTTGTC | 93 | |
| 29 | H-vector-fF29 | caggtatttctttacaattcaataatggacga | 95 | 823 |
| | H-vector-fR29 | cacagatagattgagaattttccaagcctt | 96 | |
| 30 | H-vector-fF30 | gatgttgtattagtccattctcacggtgct | 97 | 1364 |
| | H-vector-fR30 | agccaagaaataaagacaagtttctacaaccac | 98 | |

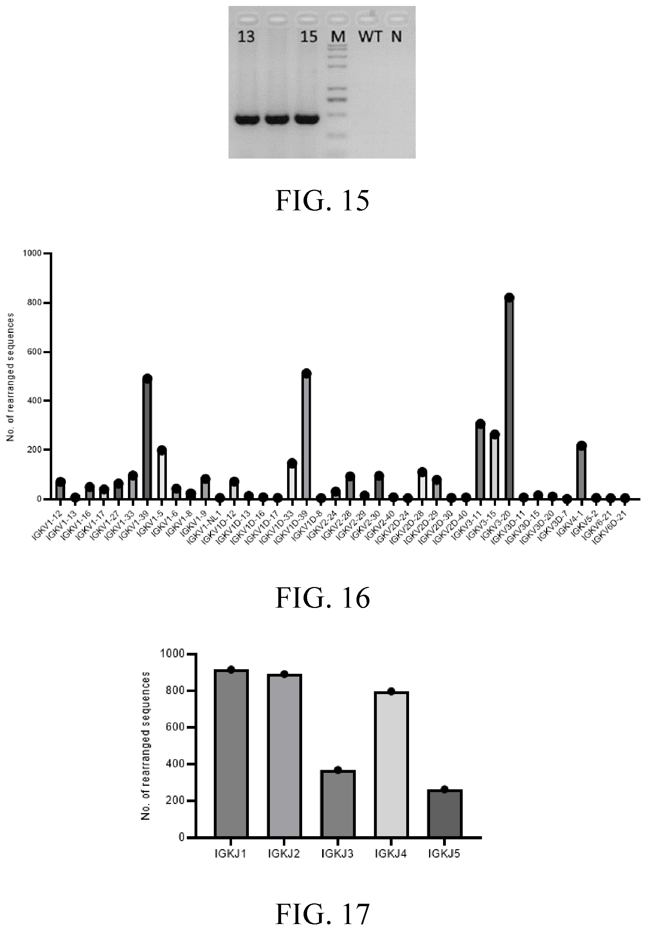

Positive cells for IGH vector1 targeting were further transfected with a transposase expression plasmid, and a screening marker fragment was deleted through a transposition mechanism. By PCR identification, it was confirmed that the resistance markers of clones 13 to 15# were correctly deleted. An identification solution for deletion of the resistance markers is shown in Table 13, and the PCR identification results of deletion of the IGH vector1 resistance markers are shown in FIG. 15.

**Table 13 Identification solution for deletion of resistance markers**

| **Serial No.** | **Primer name** | **Primer sequence** | **SEQ ID NO** | **Product size** |
|---|---|---|---|---|
| (1) FIAU | H-vector1-FIAU-tF1 | AACAAACCAGGGGTCTTAGTGATG | 99 | FIAU=485 bp |
| | H-vector1-FIAU-tR1 | GGCTAGATGCCTTTCTCCCTTGAC | 100 | |

Clones with the IGH vector1-FIAU correctly targeted were subjected to quality control by karyotype Q-PCR array analysis. The results show that the clones meet a quality control standard.

The ES cells passed the quality control by PCR and karyotype Q-PCR array analysis were injected into mouse blastocyst cavities according to a method in "Manipulating the Mouse Embryo: A Laboratory Manual" to obtain F0 chimeric mice. The chimeric mice were bred with BALB/c mice, and after genotype identification and functional validation, partially human IGH gene humanized mice were finally obtained.

### Example 4: Selection of light chain VJ regions: non-rearranged VJ and rearranged VJ

By collecting data based on the IMGT/GeneFrequency database published on the Web by LIGM (University of Montpellier, CNRS) in 2003, the frequency of usage of the V region and the frequency of usage of the J region of the human immunoglobulin light chain (IGK) were statistically analyzed, as shown in FIG. 16 and FIG. 17, and Table 14 and Table 15.

**Table 14: Selected human IGK V regions and frequencies of usage thereof published in IMGT (only frequencies greater than 100 are listed)**

| Introduced human V | IMGT/GeneFrequency |
|---|---|
| IGKV1-39 | **491** |
| IGKV1-5 | **198** |
| IGKV1D-33 | **146** |
| IGKV1D-39 | **512** |
| IGKV2D-28 | **111** |
| IGKV3-11 | **306** |
| IGKV3-15 | **263** |
| IGKV3-20 | **821** |
| IGKV4-1 | **217** |
| ... | ... |

**Table 15 Selected human IGK J regions and frequencies of usage thereof published in IMGT**

| Introduced human J | IMGT/GeneFrequency |
|---|---|
| IGKJ1 | **914** |
| IGKJ2 | **891** |
| IGKJ3 | **369** |
| IGKJ4 | **797** |
| IGKJ5 | **263** |

One of 9 IGK light chain V region genes, namely IGKV1-39, IGKV1-5, IGKV1D-33, IGKV1D-39, IGKV1-39, IGKV2D-28, IGKV3-11, IGKV3-15, and IGKV3-20, with high utilization rates in IMGT data analysis was selected and inserted downstream of the mouse-derived immunoglobulin constant region to prepare a series of mouse models.

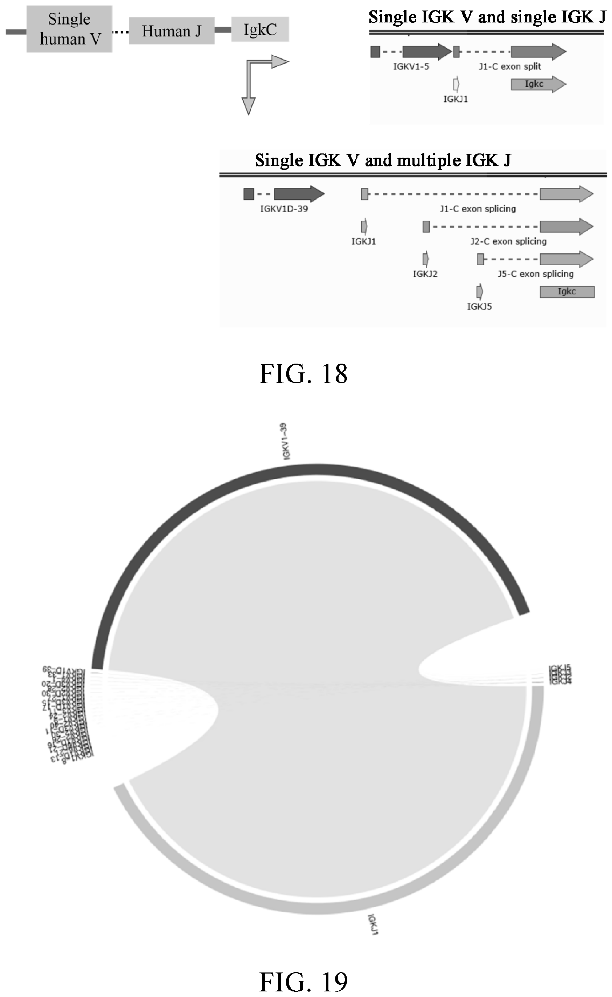

Due to relatively high frequencies of usage of the J regions of the human immunoglobulin light chains (IGK) in these mouse models, and the occurrence of usage of different J regions in currently published antibody drugs, the mouse models prepared in the present application were not subjected to human-derived IGK J region limitation. Some of the mice had all the human IGK J regions, while others had only one human IGK J region, as shown in FIG. 18. For example, Example 3 and Example 5 listed a mouse preparation solution using IGK1-39/J1-J2-J5 insertion and data on the usage of the light chain variable regions of the IGK1-39/J1 mouse B cells. For another example, in Example 7, the mice obtained in the present application using IGKV4-1/J1 insertion was selected for antibody discovery. For another example, in Example 8, the mice obtained in the present application using IGKV4-1/J1-J2-J3-J4-J5 insertion was selected for antibody discovery.

### Example 5: Light chain expression sequencing analysis without affecting diversity of heavy chains

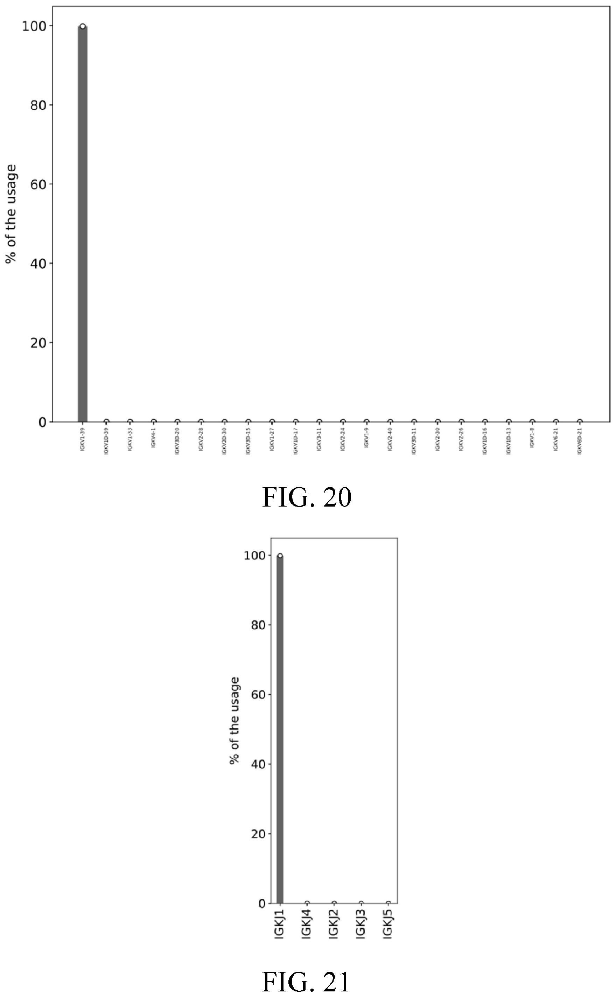

The spleens of humanized mice carrying IGK1-39 and IGKJ1 were collected, and the usage degree of the immunoglobulin light chain variable regions in spleen B cells was detected by BCR sequencing. The BCR sequencing results are shown in FIG. 19 to FIG. 21. The results show that IGK1-39 and IGKJ1 inserted downstream of the mouse-derived immunoglobulin light chain locus can be correctly transcribed and expressed as BCR variable regions, and have uniqueness. Almost all B cells used the inserted single human light chain variable regions V region and J region. The data demonstrate the success of the strategy proposed in the present application to insert only one human IGKV variable region downstream of the mouse-derived immunoglobulin light chain locus.

The frequencies of usage of the human immunoglobulin light chain variable V regions have differences under natural conditions, which may be due to the physical arrangement of the spatial distance among the V region, the J region, and the C region in the human genome. The strategy of the present application uses a single IGKV, which avoids the problem of the usage degree of the light chain V regions, so that all inserted human IGKVs can be used in the living biological system of the mouse, and a sufficient expression level is obtained. The solution of the present application may not affect the expression of the V regions due to the frequency of usage thereof in nature.

### Example 6: Normal development of humanized mouse immune cells

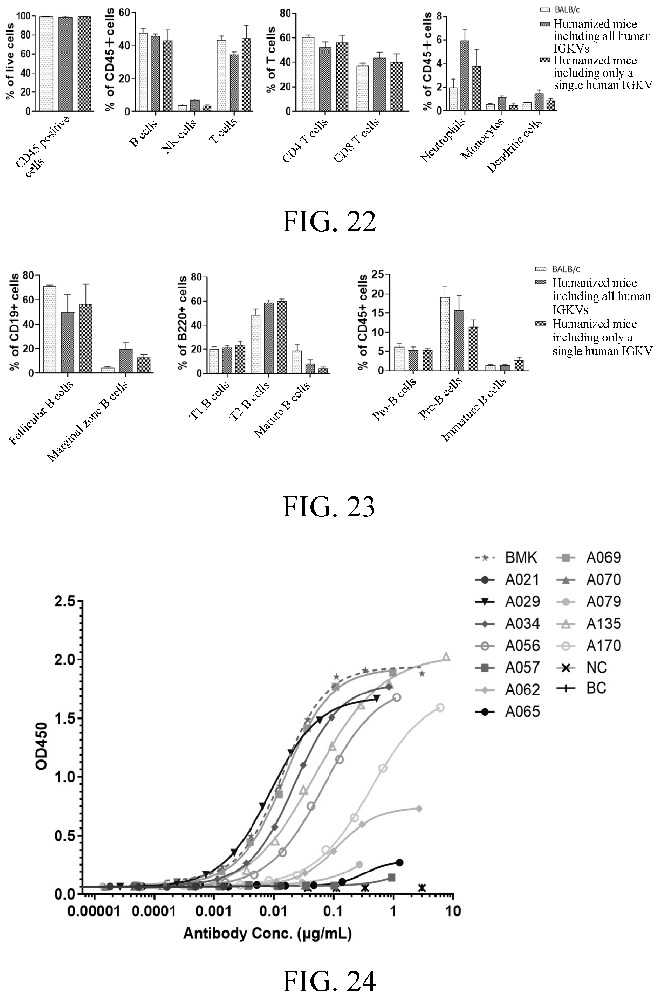

Flow cytometry was used for detecting the development of immune-related cells, especially B cells, in the spleen and bone marrow of humanized mice including only a single human light chain variable region V gene, as well as wild-type Balb/c and humanized mice including all human light chain V genes. The results, as shown in FIG. 22 and FIG. 23, demonstrate that the proportions of lymphocytes and myeloid cells in the spleen are normal, and the development of B cells in the spleen and bone marrow is normal.

### Example 7: High frequency usage of heavy chains and high frequency usage of light chains in phage library construction

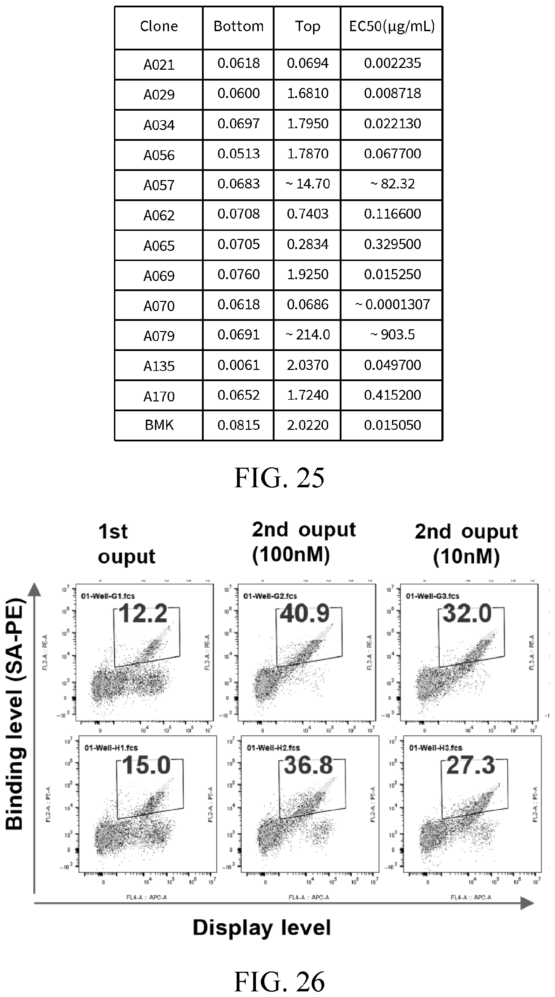

Based on the humanized mice carrying the IGK4-1 and IGKJ1 obtained by the solution of the present application, a commercial phage display technique was used for discovering antibody molecules in mice immunized with a commercial human PD1 (hPD1) recombinant protein. Table 16 shows data of the phage library established using the mice, with a library capacity of up to 10⁸. Through multiple rounds of antigen antibody affinity selection, a total of 518 clones were selected, and 183 positive clones binding to human antigens were further screened. After sequence analysis of these 183 positive clones binding to human antigens, 50 Unique antibody molecules with heavy chain sequence diversity were obtained, as shown in Table 17. The light chain variable regions of these molecules were all IGK4-1 and IGKJ1 carried by the mice. Further, the antibody affinity of 12 molecules was determined using ELISA, BLI, and the like, and the results show that high-affinity antibodies that are close to the affinity of BMK (marketed antibody drug Pembrolizumab) targeting PD1 exist in these common light chain molecules. The results are shown in FIG. 24 and FIG. 25.

These results demonstrate that the humanized mice can produce high-affinity antibodies, and the sources of the light chain variable regions of these antibodies are consistent.

**Table 16. Phage library data established using humanized mice carrying IGK4-1 and IGKJ1 and immunized with hPD1**

| **Item** | **Criteria** | **Sample** |
|---|---|---|
| **Library size (cfu)** | N/A | 9.33×10⁸ |
| **Correct expression rate (%)** | >90% | 90.48% |
| **Unique sequence (%)** | >80% | 84.21% |
| **Effective size (cfu)** | N/A | 7.11×10⁸ |

**Table 17. Heavy chain sequence diversity of 18 screened Unique antibody molecules**

| **Clone** | **VH Germline** | **VL Germline** |
|---|---|---|
| A005 | IGHV3-11*01 | IGKV4-1/J1 |
| A012 | IGHV3-7*03 | IGKV4-1/J1 |
| A021 | IGHV3-66*01, IGHV3-66*02 | IGKV4-1/J1 |
| A022 | IGHV3-7*03 | IGKV4-1/J1 |
| A024 | IGHV3-7*03 | IGKV4-1/J1 |
| A026 | IGHV3-23*04 | IGKV4-1/J1 |
| A029 | IGHV3-11*06 | IGKV4-1/J1 |
| A034 | IGHV3-30*03, IGHV3-33*05 | IGKV4-1/J1 |
| A056 | IGHV3-7*03 | IGKV4-1/J1 |
| A057 | IGHV3-7*03 | IGKV4-1/J1 |
| A062 | IGHV3-7*03 | IGKV4-1/J1 |
| A065 | IGHV3-33*08 | IGKV4-1/J1 |
| A069 | IGHV3-11*06 | IGKV4-1/J1 |
| A070 | IGHV3-7*03 | IGKV4-1/J1 |
| A079 | IGHV3-11*05 | IGKV4-1/J1 |
| A100 | IGHV3-7*03 | IGKV4-1/J1 |
| A135 | IGHV3-11*06 | IGKV4-1/J1 |
| A170 | IGHV3-7*03 | IGKV4-1/J1 |

### Example 8: Obtaining high-affinity molecules through yeast library construction

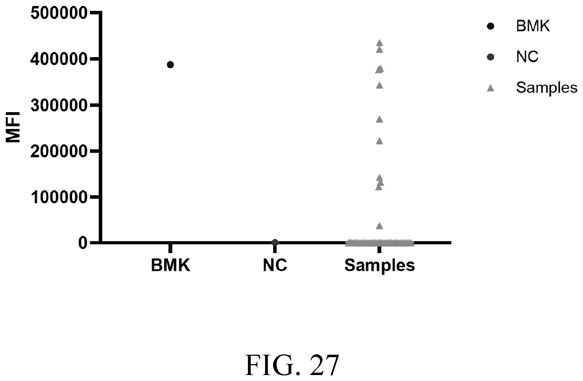

Based on the humanized mice carrying the IGKV4-1 and IGKJ1-J2-J3-J4-J5 obtained by the solution of the present application, a commercial yeast display technique was used for discovering antibody molecules in mice immunized with a commercial target protein A. Flow cytometry (FACS) selection was performed using the human-derived target protein A (Human-antigen A), a monkey-derived target protein A (Cyno-antigen A), and unrelated antigen ovalbumin (OVA). Table 18 represents establishment of a yeast display library using different immune organs (spleen and lymph node, peripheral blood PBM, and bone marrow) of different mice, and the increase in the proportion of positive antibody displaying yeast after two rounds of selection. FIG. 26 shows the enrichment of different positive antibody displaying yeast selected and obtained using the human-derived target A protein (Human-antigen A) at different concentrations. 48 clones were selected for detecting the FACS antibody antigen binding capacity at the cell supernatant level. The results (FIG. 27) show that 11 clones have the antigen binding capacity, and some clones are even better than the control BMK antibody. The top 10 clones with higher binding capacity were subjected to sequencing analysis, and the results show that these molecules have diverse CDR3 regions and diverse IGH VDJ rearrangement selections, as shown in Table 19.

The results demonstrate that the humanized mice carrying IGKV4-1 and IGKJ1-J2-J3-J4-J5 obtained using the method of the present application can produce high-affinity antibodies with heavy chain diversity and light chain constancy.

**Table 18. Selection data for yeast library construction**

| Lib ID | Tissue | V | Human-antigen A | | Cyno-antigen A | | OVA | |
|---|---|---|---|---|---|---|---|---|
| | | | 1st round | 2nd round | 1st round | 2nd round | 1st round | 2nd round |
| Mouse 1 | Spleen, lymph node | IGKV4-1 | 1.46% | 6.84% | 0.83% | 4.32% | 0.21% | 0.99% |
| Mouse 1 | PBMC, marrow | IGKV4-1 | 9.39% | 25.50% | 4.54% | 11.20% | 1.16% | 2.20% |
| Mouse 2 | Spleen, lymph node | IGKV4-1 | 12.20% | 40.90% | 3.75% | 15.90% | 0.18% | 0.16% |
| Mouse 2 | PBMC, marrow | IGKV4-1 | 15.00% | 36.80% | 3.06% | 13.30% | 0.16% | 0.26% |

**Table 19. Analysis of sequence diversity of clones with high affinity**

| **Clone ID** | **CDR H3** | **IGHV** | **IGHJ** |
|---|---|---|---|
| **>Y024-E3** | EGF*********** | IGHV3-23*04 | IGHJ4*02 |
| **>Y024-C5** | DR****** | IGHV4-30-4*09 | IGHJ4*02 |
| **>Y022-E4** | ERG********** | IGHV1-18*04 | IGHJ6*02 |
| **>Y024-B2** | ERR******* | IGHV4-30-4*09 | IGHJ4*02 |
| **>Y024-A4** | EVF********** | IGHV6-1*01 | IGHJ4*02 |
| **>Y024-C2** | GGD**************** | IGHV4-4*02 | IGHJ6*02 |
| **>Y024-A5** | SYS******* | IGHV4-34*01 | IGHJ4*03 |
| **>Y022-G3** | VLL********** | IGHV1-24*01 | IGHJ5*02 |
| **>Y021-F5** | GIT**** | IGHV3-30*03 | IGHJ6*02 |
| **>Y023-D5** | DGY********* | IGHV6-1*01 | IGHJ2*01 |

### Example 9: Preparation of partially variable region gene humanized mice

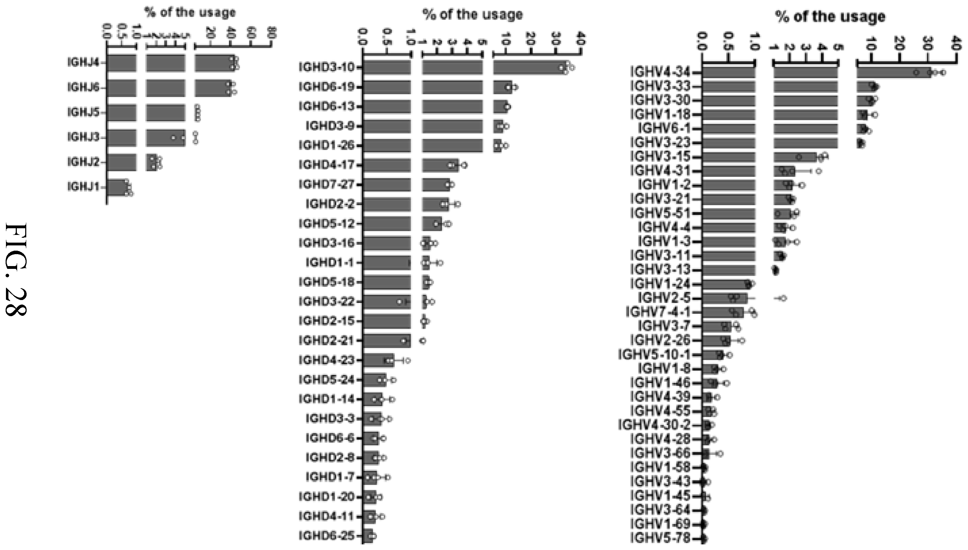

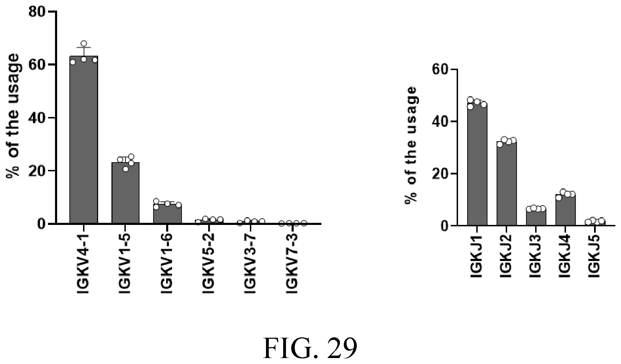

Using partially human-derived IGH-V, D, and J genes and limited human-derived IGK-V and J genes (specific gene names are listed in FIG. 19 to FIG. 20), partially VDJ gene humanized mice were obtained by the same method. The spleens of positive mice were collected and the utilization degree of human genes inserted into the spleen B cells was detected through BCR sequencing. The BCR sequencing results, as shown in FIG. 28 and FIG. 29, demonstrate that even with the insertion of partial VDJ genes, the humanized mice can still use this part of the human variable region gene.

### Example 10: The partially variable region gene humanized mice can produce immune response normally

The VDJ partially humanized mice were immunized using a commercial OVA protein, and the serum antibody ELISA titer determination was performed.

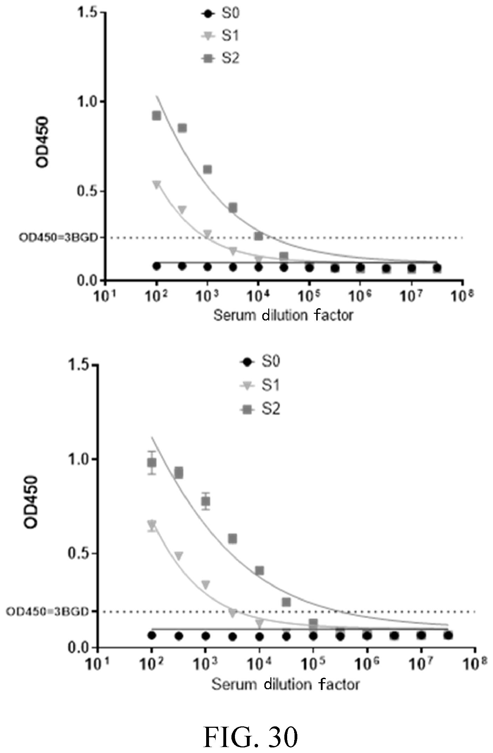

As shown in FIG. 30, the immune response of the humanized mice to the OVA antigen is comparable to that of the BALB/c mice.

## Claims

1. A method for preparing a non-human animal, the method comprising operably linking a human immunoglobulin light chain variable region gene downstream of an immunoglobulin light chain locus of the non-human animal, wherein the human immunoglobulin light chain variable region comprises only one human V region gene, and preferably, the human immunoglobulin light chain variable region gene and an endogenous immunoglobulin light chain variable region gene of the non-human animal comprise an immunoglobulin light chain constant region gene of the non-human animal therebetween.

2. The method according to claim 1, wherein the human immunoglobulin light chain variable region gene further comprises one or more human J region genes.

3. The method according to claim 2, wherein the human immunoglobulin light chain variable region comprises 2 to 5 human J region genes.

4. The method according to claim 2 or 3, wherein the only one human V region gene and the one or more human J region genes are operably linked.

5. The method according to any one of claims 1 to 4, wherein the human V region gene is selected from the group consisting of: IGKV4-1, IGKV1-5, IGKV3-11, IGKV3-15, IGKV3-20, IGKV1D-39, IGKV1-39, IGKV2D-28, and IGKV1D-33.

6. The method according to any one of claims 1 to 5, wherein the human V region gene and J region gene comprise a gene rearrangement intron sequence therebetween.

7. The method according to any one of claims 1 to 5, wherein the human V region gene and J region gene do not comprise a gene rearrangement intron sequence therebetween.

8. The method according to any one of claims 2 to 7, wherein the human J region gene is selected from the group consisting of: IGKJ5, IGKJ4, IGKJ3, IGKJ2, and IGKJ1.

9. The method according to any one of claims 2 to 8, wherein the human J region gene comprises the gene rearrangement intron sequence.

10. The method according to any one of claims 2 to 8, wherein the human J region gene does not comprise the gene rearrangement intron sequence.

11. The method according to any one of claims 1 to 10, wherein genomic integrity of an endogenous immunoglobulin light chain variable region of the non-human animal remains unchanged.

12. The method according to any one of claims 1 to 11, wherein a function of an expression regulatory element of the endogenous immunoglobulin light chain variable region gene of the non-human animal is not disrupted.

13. The method according to any one of claims 1 to 12, wherein the non-human animal comprises a complete expression regulatory element of the endogenous immunoglobulin light chain variable region.

14. The method according to any one of claims 1 to 13, wherein the non-human animal comprises a complete endogenous immunoglobulin light chain variable region gene.

15. The method according to any one of claims 1 to 14, wherein the non-human animal does not express the endogenous immunoglobulin light chain variable region.

16. The method according to any one of claims 1 to 15, wherein the endogenous immunoglobulin light chain variable region gene of the non-human animal is not expressed as an antibody light chain variable region.

17. The method according to any one of claims 1 to 16, comprising rendering a transcription direction of the human immunoglobulin light chain variable region gene to be opposite to that of the endogenous immunoglobulin light chain variable region gene.

18. The method according to any one of claims 1 to 17, wherein the human immunoglobulin light chain variable region gene and the endogenous immunoglobulin light chain variable region comprise the immunoglobulin light chain constant region gene of the non-human animal therebetween.

19. The method according to claim 18, comprising rendering a transcription direction of the immunoglobulin light chain constant region gene of the non-human animal to be opposite to that of the endogenous immunoglobulin light chain variable region gene.

20. The method according to any one of claims 1 to 19, wherein in the non-human animal, the transcription direction of the human immunoglobulin light chain variable region gene is the same as that of an immunoglobulin constant region gene of the non-human animal.

21. The method according to any one of claims 1 to 20, wherein a distance between the human immunoglobulin light chain variable region gene and the endogenous immunoglobulin light chain variable region gene is 4 Kbp to 42 Kbp.

22. The method according to any one of claims 1 to 21, wherein the non-human animal is a rodent.

23. The method according to any one of claims 1 to 22, wherein the non-human animal is a mouse.

24. The method according to claim 23, comprising inserting the human immunoglobulin light chain variable region gene between lgkc and Rpia genes at a locus of the mouse.

25. The method according to any one of claims 23 to 24, comprising inserting the human immunoglobulin light chain variable region gene between chromosome positions chr6: 70,703,738 and 70,742,704 of the mouse.

26. The method according to any one of claims 23 to 25, comprising inserting the human immunoglobulin light chain variable region gene at a chromosome position chr6: 70,706,267 of the mouse.

27. The method according to any one of claims 1 to 26, comprising enabling the human immunoglobulin light chain variable region gene to be operably linked downstream of the immunoglobulin locus of the non-human animal through site-directed recombination.

28. The method according to any one of claims 19 to 27, comprising rendering the transcription direction of the immunoglobulin light chain constant region gene of the non-human animal to be opposite to that of the endogenous immunoglobulin light chain variable region gene through genome modification.

29. A non-human animal, wherein a genome of the non-human animal comprises an operably linked human immunoglobulin light chain variable region gene downstream of an immunoglobulin light chain locus, the human immunoglobulin light chain variable region comprises only one human V region gene, and preferably, the human immunoglobulin light chain variable region gene and an endogenous immunoglobulin light chain variable region gene of the non-human animal comprise an immunoglobulin light chain constant region gene of the non-human animal therebetween.

30. The non-human animal according to claim 29, wherein the human immunoglobulin light chain variable region gene further comprises one or more human J region genes, preferably 2 to 5 human J region genes.

31. The non-human animal according to claim 30, wherein the only one human V region gene and the one or more human J region genes are operably linked.

32. The non-human animal according to any one of claims 29 to 31, wherein the human V region gene is selected from the group consisting of: IGKV4-1, IGKV1-5, IGKV3-11, IGKV3-15, IGKV3-20, IGKV1D-39, IGKV1-39, IGKV2D-28, and IGKV1D-33.

33. The non-human animal according to any one of claims 29 to 32, wherein the human V region gene and J region gene comprise a gene rearrangement intron sequence therebetween.

34. The non-human animal according to any one of claims 29 to 33, wherein the human V region gene and J region gene do not comprise the gene rearrangement intron sequence therebetween.

35. The non-human animal according to any one of claims 30 to 34, wherein the human J region gene is selected from the group consisting of: IGKJ5, IGKJ4, IGKJ3, IGKJ2, and IGKJ1.

36. The non-human animal according to any one of claims 30 to 35, wherein the human J region gene comprises the gene rearrangement intron sequence.

37. The non-human animal according to any one of claims 30 to 36, wherein the human J region gene does not comprise the gene rearrangement intron sequence.

38. The non-human animal according to any one of claims 29 to 37, wherein genomic integrity of an endogenous immunoglobulin light chain variable region remains unchanged.

39. The non-human animal according to any one of claims 29 to 38, wherein a function of an expression regulatory element of the endogenous immunoglobulin light chain variable region gene is not disrupted.

40. The non-human animal according to any one of claims 29 to 39, wherein the *genome* of the non-human animal comprises a complete expression regulatory element of the endogenous immunoglobulin light chain variable region.

41. The non-human animal according to any one of claims 29 to 40, wherein the *genome* of the non-human animal comprises a complete endogenous immunoglobulin light chain variable region gene.

42. The non-human animal according to any one of claims 29 to 41, wherein the non-human animal does not express the endogenous immunoglobulin light chain variable region.

43. The non-human animal according to any one of claims 29 to 42, wherein the endogenous immunoglobulin light chain variable region gene is not expressed as an antibody light chain variable region.

44. The non-human animal according to any one of claims 29 to 43, wherein a transcription direction of the human immunoglobulin light chain variable region gene is opposite to that of the endogenous immunoglobulin light chain variable region gene.

45. The non-human animal according to any one of claims 29 to 44, wherein the human immunoglobulin light chain variable region gene and the endogenous immunoglobulin light chain variable region comprise the immunoglobulin light chain constant region gene of the non-human animal therebetween.

46. The non-human animal according to claims 29 to 44, wherein a transcription direction of the immunoglobulin light chain constant region gene is opposite to that of the endogenous immunoglobulin light chain variable region gene.

47. The non-human animal according to any one of claims 29 to 46, wherein the transcription direction of the human immunoglobulin light chain variable region gene is the same as that of an immunoglobulin constant region gene of the non-human animal.

48. The non-human animal according to any one of claims 29 to 47, wherein a distance between the human immunoglobulin light chain variable region gene and the endogenous immunoglobulin light chain variable region gene is 4 Kbp to 42 Kbp.

49. The non-human animal according to any one of claims 29 to 48, wherein the non-human animal is a rodent.

50. The non-human animal according to any one of claims 29 to 49, wherein the non-human animal is a mouse.

51. The non-human animal according to claim 50, wherein the non-human animal comprises the human immunoglobulin light chain variable region gene between lgkc and Rpia genes at a locus of the non-human animal.

52. The non-human animal according to any one of claims 50 to 51, wherein the non-human animal comprises the human immunoglobulin light chain variable region gene between chromosome positions chr6: 70,703,738 and 70,742,704.

53. The non-human animal according to any one of claims 50 to 52, wherein the non-human animal comprises the human immunoglobulin light chain variable region gene at a chromosome position chr6: 70,706,267.

54. A non-human animal cell, wherein a genome of the non-human animal cell comprises an operably linked human immunoglobulin light chain variable region gene downstream of an immunoglobulin light chain locus, and the human immunoglobulin light chain variable region comprises only one human V region gene.

55. A method for preparing an antibody specifically binding to an antigen, comprising immunizing the non-human animal according to any one of claims 29 to 53 with the antigen.

56. A method for preparing a sample, comprising: exposing the non-human animal according to any one of claims 29 to 53 to an antigen; and collecting the sample from the non-human animal.

57. The method according to claim 56, wherein the sample comprises immune cells.

58. The method according to any one of claims 56 to, wherein the sample comprises B cells.

59. The method according to any one of claims 56 to 58, wherein the sample is derived from bone marrow, spleen tissues, lymph nodes, spleen cells, or peripheral lymphocytes.

60. A genetically modified non-human animal genome, comprising an operably linked human immunoglobulin light chain variable region gene downstream of an immunoglobulin light chain locus, wherein the human immunoglobulin light chain variable region comprises only one human V region gene, and preferably, the human immunoglobulin light chain variable region gene and an endogenous immunoglobulin light chain variable region gene of the non-human animal comprise an immunoglobulin light chain constant region gene of the non-human animal therebetween.

61. The non-human animal genome according to claim 60, wherein the human immunoglobulin light chain variable region gene further comprises one or more human J region genes, preferably 2 to 5 human J region genes.

62. The non-human animal genome according to claim 61, wherein the only one human V region gene and the one or more human J region genes are operably linked.

63. The non-human animal genome according to any one of claims 60 to 62, wherein the human V region gene is selected from the group consisting of: IGKV4-1, IGKV1-5, IGKV3-11, IGKV3-15, IGKV3-20, IGKV1D-39, IGKV1-39, IGKV2D-28, and IGKV1D-33.

64. The non-human animal genome according to any one of claims 60 to 63, wherein the human V region gene and J region gene comprise a gene rearrangement intron sequence therebetween.

65. The non-human animal genome according to any one of claims 60 to 64, wherein the human V region gene and J region gene do not comprise the gene rearrangement intron sequence therebetween.

66. The non-human animal genome according to any one of claims 61 to 65, wherein the human J region gene is selected from the group consisting of: IGKJ5, IGKJ4, IGKJ3, IGKJ2, and IGKJ1.

67. The non-human animal genome according to any one of claims 61 to 65, wherein the human J region gene comprises the gene rearrangement intron sequence.

68. The non-human animal genome according to any one of claims 61 to 67, wherein the human J region gene does not comprise the gene rearrangement intron sequence.

69. The non-human animal genome according to any one of claims 61 to 68, wherein genomic integrity of an endogenous immunoglobulin light chain variable region remains unchanged.

70. The non-human animal genome according to any one of claims 60 to 69, wherein a function of an expression regulatory element of the endogenous immunoglobulin light chain variable region gene is not disrupted.

71. The non-human animal genome according to any one of claims 60 to 70, wherein the non-human animal genome comprises a complete expression regulatory element of the endogenous immunoglobulin light chain variable region.

72. The non-human animal genome according to any one of claims 60 to 71, wherein the non-human animal genome comprises a complete endogenous immunoglobulin light chain variable region gene.

73. The non-human animal genome according to any one of claims 60 to 72, wherein the non-human animal genome does not express the endogenous immunoglobulin light chain variable region.

74. The non-human animal genome according to any one of claims 60 to 73, wherein the endogenous immunoglobulin light chain variable region gene is not expressed as an antibody light chain variable region.

75. The non-human animal genome according to any one of claims 60 to 74, wherein a transcription direction of the human immunoglobulin light chain variable region gene is opposite to that of the endogenous immunoglobulin light chain variable region gene.

76. The non-human animal genome according to any one of claims 60 to 75, wherein the human immunoglobulin light chain variable region gene and the endogenous immunoglobulin light chain variable region comprise the immunoglobulin light chain constant region gene of the non-human animal therebetween.

77. The non-human animal genome according to any one of claims 60 to 76, wherein a transcription direction of the immunoglobulin light chain constant region gene is opposite to that of the endogenous immunoglobulin light chain variable region gene.

78. The non-human animal genome according to any one of claims 60 to 77, wherein the transcription direction of the human immunoglobulin light chain variable region gene is the same as that of an immunoglobulin constant region gene of the non-human animal.

79. The non-human animal genome according to any one of claims 60 to 78, wherein a distance between the human immunoglobulin light chain variable region gene and the endogenous immunoglobulin light chain variable region gene is 4 Kbp to 42 Kbp.

80. The non-human animal genome according to any one of claims 60 to 79, wherein the non-human animal is a rodent.

81. The non-human animal genome according to any one of claims 60 to 80, wherein the non-human animal is a mouse.

82. The non-human animal genome according to claim 81, wherein the non-human animal genome comprises the human immunoglobulin light chain variable region gene between lgkc and Rpia genes.

83. The non-human animal genome according to any one of claims 81 to 82, wherein the non-human animal genome comprises the human immunoglobulin light chain variable region gene between chromosome positions chr6: 70,703,738 and 70,742,704.

84. The non-human animal genome according to any one of claims 81 to 83, wherein the non-human animal genome comprises the human immunoglobulin light chain variable region gene at a chromosome position chr6: 70,706,267.

85. An engineered chromosome, comprising the non-human animal genome according to any one of claims 60 to 84.

86. A cell, comprising the engineered chromosome according to claim 85.
